# EUROPEAN PATENT SPECIFICATION

(11) **EP 3 735 260 B1**
(45) Date of publication and mention of the grant of the patent: **04.03.2026**
(21) Application number: 19736089.4
(22) Date of filing: 04.01.2019
(51) Int. Cl.: A61K 38/00, A61K 38/03, A61K 38/07, A61K 38/08, A61K 38/10, A61K 38/16, A61P 3/10, C07K 14/705, G01N 33/68

(54) **THERAPEUTIC PEPTIDES AND METHODS FOR TREATING AUTOIMMUNE RELATED DISEASE**
THERAPEUTISCHE PEPTIDE UND VERFAHREN ZUR BEHANDLUNG VON AUTOIMMUNERKRANKUNGEN
PEPTIDES THÉRAPEUTIQUES ET MÉTHODES DE TRAITEMENT DE MALADIES LIÉES À L'AUTO-IMMUNITÉ

(30) Priority: 05.01.2018 US 201862614262 P; 08.11.2018 US 201816184129
(43) Date of publication of application: 11.11.2020
(73) Proprietor: OP-T LLC, Denver, Colorado 80206 (US)
(72) Inventor: WAGNER, JR., David Hal, Denver, Colorado 80220 (US); YUSSMAN, Martin Glenn, Denver, Colorado 80128 (US); HENRY, Charles W., Denver, Colorado 80209 (US)
(74) Representative: ZBM Patents - Zea, Barlocci & Markvardsen
(86) International application number: PCT/US2019/012425
(87) International publication number: WO 2019/136307

(56) References cited:
- WO-A2-2015/148389
- US-A1- 2007 243 259
- US-A1- 2011 177 556
- US-A1- 2011 229 495
- US-A1- 2017 355 747
- POGGI M ET AL: "The inflammatory receptor CD40 is expressed on human adipocytes: contribution to crosstalk between lymphocytes and adipocytes", DIABETOLOGIA, SPRINGER, BERLIN, DE, vol. 52, no. 6, 31 January 2009 (2009-01-31), pages 1152 - 1163, XP019698542, ISSN: 1432-0428
- OCEL JEFF ET AL: "Acute exercise induces GLUT4 translocation in skeletal muscle of normal human subjects and subjects with type 2 diabetes Cardiac CT volumetric and diastolic function assessment View project Hernias View project", DIABETES, 1 June 1999 (1999-06-01), XP055843841, Retrieved from the Internet <URL:https://www.researchgate.net/publication/12968138_Acute_exercise_induces_GLUT4_translocation_in_skeletal_muscle_of_normal_human_subjects_and_subjects_with_type_2_diabetes> [retrieved on 20210922], DOI: 10.2337/diabetes.48.5.1192.Source:
- SHI WEIBIN ET AL: "Ldlr-Deficient Mice with an Atherosclerosis-Resistant Background Develop Severe Hyperglycemia and Type 2 Diabetes on a Western-Type Diet", BIOMEDICINES, vol. 10, no. 6, 16 June 2022 (2022-06-16), pages 1429, XP093021696, DOI: 10.3390/biomedicines10061429
- SEIJKENS ET AL.: "CD 40- CD 40L: linking pancreatic, adipose tissue and vascular inflammation in type 2 diabetes and its complications", DIAB VASC DIS RES, vol. 10, 10 September 2012 (2012-09-10), pages 115 - 122, XP055622225
- SAFAR ET AL.: "ATHEROSCLEROSIS, LARGE ARTERIES AND CARDIOVASCULAR RISK", vol. 44, 12 August 2008, ISBN: 978-3-8055-8176-9, article RACHEL H. MACKEY, LAKSHMI VENKITACHALAM, KIM SUTTON-TYRRELL: "Calcifications, arterial stiffness, and atherosclerosis", pages: 234 - 244, XP009522000
- GARLICHS ET AL.: "Upregulation of CD 40 and CD 40 ligand ( CD 154) in patients with moderate hypercholesterolemia", CIRCULATION, vol. 104, 13 November 2001 (2001-11-13), pages 2395 - 2400, XP055622229
- KUO HUEY-LIANG, HUANG CHIU-CHING, LIN TZE-YI, LIN CHING-YUANG: "IL -17 and CD 40 ligand synergistically stimulate the chronicity of diabetic nephropathy", NEPHROLOGY DIALYSIS TRANSPLANTATION, vol. 33, no. 3, 1 March 2017 (2017-03-01), pages 248 - 256, XP009521993, ISSN: 0931-0509, DOI: 10.1093/ndt/gfw397
- IEZZI ET AL.: "CD 40- CD 40L cross-talk integrates strong antigenic signals and microbial stimuli to induce development of IL -17-producing CD 4+ T cells", PROC NATL ACAD SCI USA, vol. 106, 9 January 2009 (2009-01-09), pages 876 - 881, XP007915728, DOI: 10.1073/pnas.0810769106
- WINER ET AL.: "B cells promote insulin resistance through modulation of T cells and production of pathogenic IgG antibodies", NAT MED, vol. 17, 17 April 2011 (2011-04-17), pages 610 - 617, XP055622231

## Description

The present disclosure relates to peptides for use in methods of preventing, reducing, treating and/or reversing of type 2 diabetes mellitus, via administration of a therapeutically effective amount the corresponding peptide, wherein the peptide is a CD40-binding peptide that inhibits, the interaction of CD40 and CD154.

### Background

Autoimmune diseases are conditions arising from an abnormal immune response to a normal body part. More than 80 diseases occur because of the immune system attacking the body's own organs, tissues, and cells. Type 1 diabetes, rheumatoid arthritis, systemic lupus erythematosus, and inflammatory bowel disease are common autoimmune diseases that affect a wide range of people across entire populations. Significantly, the autoimmune disorders mentioned above afflict substantial number of people affecting their daily lives and routines and require significant monetary and healthcare resources, time and care from healthcare providers.

According to the World Health Organization (WHO), an estimated 17.7 million people died from cardiovascular diseases (CVDs) in 2015, which represented 31% of all global deaths. CVDs, may also be known as heart and blood vessel disease, and includes numerous problems, many of which are related to a process called atherosclerosis. Moreover, according to the Healthcare Cost and Utilization Project (HCUP) which is sponsored by the Agency for Healthcare Research and Quality (AHRQ), in 2011, coronary atherosclerosis alone accounted for more than $10.4 billion in hospital costs. Accordingly, in 2011, coronary atherosclerosis alone was one of the ten most expensive conditions for inpatient hospitalizations in the United States.

### Cardiovascular Disease and Atherosclerosis

Atherosclerosis is defined by arterial plaque formation that may lead to heart attack and stroke. Arterial plaque formation is caused by the deposition of cells, substances, waste products, and cellular debris including, but not limited to: cholesterol, dead cells, dendritic cells, foam cells, macrophages, mast cells, monocytes, smooth muscle cells, T-cells, collagen, calcium, and fibrin. Inflammatory changes within the arterial wall and plaque may play a crucial and causative role in atherosclerotic disease development. Consequently, the concept of atherosclerosis as an autoimmune and inflammatory disease has been investigated; however, a therapeutic control has not been established. The importance of controlling inflammation is highlighted by current clinical trials targeting other aspects of autoimmune, inflammation, and cardiovascular disease and death.

For example, CIRT (Cardiovascular Inflammation Reduction Trial) is attempting to use methotrexate to target interleukin-6 (IL-6) to test whether methotrexate will reduce rates of myocardial infarction, stroke, and cardiovascular death among patients with coronary artery disease patients with type 2 diabetes. Another example includes, CANTOS (Canakinumab Anti-inflammatory Thrombosis Outcomes Study) which is studying whether canakinumab can block the pro-inflammatory cytokine interleukin - 1β (IL-1β) to reduce rates of recurrent myocardial infarction, stroke, and cardiovascular death rates in heart attack patients who remain at a high risk. This risk is demarcated by elevated levels of the inflammatory biomarker high sensitivity C-reactive protein (hsCRP). These studies acknowledge that inflammation plays a critical role in atherothrombosis and atherosclerosis; however, these studies also recognize that is unknown whether inhibition of inflammation per se will lower vascular event rates.

Mammalian and human atherosclerotic lesions are characterized as a chronic inflammatory-fibroproliferative disease of the blood vessel wall containing monocytes, macrophages, endothelial cells, smooth muscle cells, platelets, and T-cells. Each of these cell types can express either or both of the CD40/CD154 costimulatory pair. This dyad is responsible for enhancing the immune response and may contribute to many chronic inflammatory diseases including rheumatoid arthritis, multiple sclerosis, and type 1 diabetes (T1D). However, no viable therapy exists for this highly atherogenic dyad.

Inflammation may occur when inflammatory cells, such as neutrophils, eosinophils, basophils, mast cells, macrophages, platelets, and endothelial cells, respond to inflammatory events or harmful stimuli, such as, invading microorganisms, damages cells, or other irritants. The body's inflammatory response is beneficial because for example, in the case of invading microorganisms, the inflammatory response is an important step in localizing the infecting agent for removal by the immune system. However, in autoimmunity there is no infection, yet severe inflammation is present or persistent. The inflammation in this case, referred to as aseptic chronic inflammation (ACI), is detrimental since it destroys normal tissues. The results of this aseptic inflammation are life-altering and in some cases life-threatening. Moreover, as with acute inflammation, this process is mediated by immune cells, including T-cells.

A major concern for modern medicine is how to control ACI such as that which occurs during autoimmune diseases, as well as how to control acute inflammation resulting from trauma. Inflammation, both chronic and acute, leads to tissue degeneration and eventual loss of function of major organs. ACI is not limited to a single disease, but is instrumental in numerous autoimmune diseases, including, but not limited to: type 1 diabetes (T1D), multiple sclerosis (MS), systemic lupus erythematosus (SLE), rheumatoid arthritis (RA), Crohn's disease, inflammatory bowel disease (IBS), chronic obstructive pulmonary disease (COPD) including types of autoimmune asthma, atherosclerosis, vasculitis, hypertension, thyroiditis including Hashimoto's and Graves diseases, primary biliary cirrhosis, Paget's disease, Addison's disease, acute respiratory distress syndrome (ARDS), acute lung injury, and aseptic chronic inflammation (ACI) associated with organ transplantation.

Autoimmune disorders are classified into two types: organ-specific (directed mainly at one organ) and non-organ-specific (widely spread throughout the body). Examples of organ-specific autoimmune disorders are insulin-dependent Type 1 diabetes (T1D) which affects the pancreas; Hashimoto's thyroiditis and Graves' disease, which affect the thyroid gland; pernicious anemia, which affects the blood; Addison's disease, which affects the adrenal glands; chronic active hepatitis, which affects the liver; myasthenia gravis which affects the muscle; and multiple sclerosis (MS), which affects tissue of the nervous system. An example of a non-organ-specific autoimmune disorders is rheumatoid arthritis (RA). Autoimmune diseases are often chronic, debilitating, and life-threatening. The National Institutes of Health (NIH) estimates that up to 23.5 million Americans suffer from autoimmune disease and that the prevalence is rising. It has been estimated that autoimmune diseases are among the ten leading causes of death among women in all age groups up to 65 years.

Acute inflammation, as observed during trauma or sepsis, is also immune cell mediated. While a comprehensive, complete, and exhaustive list of the molecular mediators in this process have not yet been identified, a prominent role for T-cells, lymphocytes, neutrophils, macrophages, monocytes, neutrophils, eosinophils, basophils, mast cells, and other inflammatory cells is strongly implicated. Therefore, a process to modulate these cell types may control the inflammatory response.

### Type 2 Diabetes

The CD40-CD154 dyad constitutes a major inflammatory pathway (Schonbeck U, et al. Cell Mol. Life Sci. (2001) 58(1):4-43) that plays a significant role in type 1 diabetes (Waid DM. et al. Clin. Immunol. (2007) 124(2):138-148). Type 2 diabetes (T2D) has historically and scientifically been primarily categorized as a metabolic disorder; however, type 2 diabetes is in the process of being redefined as an autoimmune disease rather than just a metabolic disorder (Winer, D, et al. Nature Medicine (2011) 17:610-617).

According to the United States Centers for Disease Control National Diabetes Statistics Report for 2017 (available at https://www.cdc.gov/diabetes/pdfs/data/statistics/national-diabetes-statistics-report.pdf), an estimated 30.3 million people of all ages - or 9.4% of the U.S. population had diabetes in 2015. An estimated 23 million people - or 7.2% of the U.S. population had been diagnosed with diabetes mellitus, and about 95% of those diagnosed with diabetes have type 2 diabetes. Based on fasting plasma glucose levels, one third to one half of cases of type 2 diabetes are undiagnosed and untreated (Harris MI, Diabetes Care. (1998) 21 [Suppl. 3: C11-C14]; Howard BV, et al., Circulation. 2002; 105:e132-e137; Grundy SM, et al., Circulation. 1999; 100: 1134-1146).

Although type 2 diabetes most often develops in people over the age of 45, type 2 diabetes increased 21% in American youth from 2001 to 2009 and a large study called SEARCH for Diabetes in Youth found that newly diagnosed cases of Type 2 diabetes in children and teens increased by about 4.8 percent in each year of the study's period between 2002 and 2012 ("Rates of new diagnosed cases of type 1 and type 2 diabetes on the rise among children, teens" National Institutes of Health, April 13, 2017, available at https://www.nih.gov/news-events/news-releases/rates-new-diagnosed-cases-type-1-type-2-diabetes-rise-among-children-teens).

Major comorbidities complicate diabetes, the most common being cardiovascular disease (70.4 per 1,000 persons) including those with ischemic heart disease and stroke. Overall, for the year 2012, the American Diabetes Association estimates that the total direct and indirect estimated cost in the United States was $245 billion, including $176 billion in direct medical costs and $69 billion in reduced productivity (Yang, W. American Diabetes Association, 2013, Diabetes Care, 36 (4): 1033-46).

Both type 1 and type 2 diabetes are powerful and independent risk factors for coronary artery disease (CAD), stroke, and peripheral arterial disease (Schwartz CJ, et al. Diabetes Care. (1992) 15:1156-1167; Stamler, J. et al. Diabetes Care. (1993) 16:434-444; Beckman JA, et al., Diabetes and atherosclerosis: epidemiology, pathophysiology, and management. JAMA (2002) 287:2570-2581). Atherothrombosis accounts for 65% to 80% of all deaths among North American patients with diabetes, compared with about 33% of all deaths in the general North American population (American Diabetes Association. Diabetes Care. (1993) 16:72-78). Therefore, a therapeutic regimen that is effective and can be tolerated for long periods of time would be beneficial to individuals and from a public health perspective. An ideal anti-diabetic agent may be an agent which corrects hyperglycemia, prevents macrovascular complications, and corrects the pathophysiological disturbances responsible for Type 2 Diabetes ("T2D"). Insulin resistance is basic to T2D, but β-cell failure eventually occurs with imbalance between insulin resistance and insulin secretion being a further complication. Therefore, therapeutically beneficial treatment approaches may aim to reverse insulin resistance and improve β-cell function.

T2D often conincides with obesity however genetic and environmental factors recently have been described as disease contributors (Comuzzie AG, Best Pract. Res. Clin. Endocrinol. Metab. (2002) 16(4):611-21. PubMed PMID: 12468410; van Tilberg J., et al. J. Med. Genet. (2001) 38(9):569-78. Pub Med PMID: 11546824; PMCID: PMC 1734947). Moreover, additional research has emerged that indicates that T2D, like T1D, has prominent inflammation component that is a contributing and/or driving factor of the T2D disease commencement, development and progression. The CD40-CD154 inflammatory dyad may act as a molecular driver to propel autoimmune inflammation and influence excessive levels of the dyad may be an unappreciated but contributing factor to T2D. (Hseih CJ, et al., Cir. J. (2009) 73(5) 948-54; Kutlu M, et al., Clin. Invest. Med. (2009) 32(6): E244; Santilli F, et al., J. Am. Coll. Cardiol. (2006) 47(2):391-7; Santini, E, et al. J. Endocrinol Invest. 2008; 31(7):660-5; Varo N, et al., Circulation. 2003; 107(21):2664-9).

Generally, inflammation may occur when inflammatory cells, such as neutrophils, eosinophils, basophils, mast cells, macrophages, platelets, endothelial cells, and lymphocytes, including but not limited to T cells and B cells respond to inflammatory events or harmful stimuli, such as, invading microorganisms, damaged cells, or other irritants. The body's inflammatory response is beneficial because, for example, in the case of invading microorganisms, the inflammatory response is an important step in localizing the infecting agent for removal by the immune system. However, in autoimmunity there is no infection, yet severe inflammation is present or persistent. The inflammation in this case, referred to as aseptic chronic inflammation (ACI), is detrimental since it destroys normal tissues. The results of this aseptic inflammation are life-altering and in some cases life-threatening. Moreover, as with acute inflammation, this process is mediated by immune cells, including T-cells.

A major concern for modern medicine is how to control ACI such as that which occurs during autoimmune diseases, as well as how to control acute inflammation resulting from trauma. Inflammation, both chronic and acute, leads to tissue degeneration and eventual loss of function of major organs. ACI is not limited to a single disease, but is instrumental in numerous autoimmune diseases, including, but not limited to: type 1 diabetes (T1D), multiple sclerosis, systemic lupus erythematosus, rheumatoid arthritis, Crohn's disease, inflammatory bowel disease, chronic obstructive pulmonary disease including types of autoimmune asthma, atherosclerosis, vasculitis, hypertension, thyroiditis including Hashimoto's and Graves diseases, primary biliary cirrhosis, Paget's disease, Addison's disease, acute respiratory distress syndrome, acute lung injury, and ACI associated with organ transplantation.

Autoimmune disorders are classified into two types: organ-specific (directed mainly at one organ) and non-organ-specific (widely spread throughout the body). Examples of organ-specific autoimmune disorders are insulin-dependent Type 1 diabetes (T1D) which affects the pancreas; Hashimoto's thyroiditis and Graves' disease, which affect the thyroid gland; pernicious anemia, which affects the blood; Addison's disease, which affects the adrenal glands; chronic active hepatitis, which affects the liver; myasthenia gravis which affects the receptors at the junction between nerves and muscles; and multiple sclerosis, which affects tissue of the nervous system. An example of a non-organ-specific autoimmune disorders is rheumatoid arthritis. Autoimmune diseases are often chronic, debilitating, and life-threatening. The National Institutes of Health (NIH) estimates that up to 23.5 million Americans suffer from autoimmune disease and that the prevalence is rising. It has been estimated that autoimmune diseases are among the ten leading causes of death among women in all age groups up to 65 years.

Acute inflammation, as observed during trauma or sepsis, is also immune cell mediated. While a comprehensive, complete, and exhaustive list of the molecular mediators in this process have not yet been identified, a prominent role for T-cells, lymphocytes, neutrophils, macrophages, monocytes, neutrophils, eosinophils, basophils, mast cells, and other inflammatory cells is strongly implicated. Therefore, a process to modulate these cell types may control the inflammatory response.

A unique T cell subset has been shown to be instrumental in the development of autoimmune disease. These cells are phenotypically characterized as CD4loCD40+ (Waid, D.M., et al., Eur. J. of Immunol., 34:1488, 2004; Vaitaitis, G.M., et al., Cutting Edge, J. Immunol., 170:3455, 2003; Wagner, D.H., Jr., et al., Proc. Nat 'l. Acad. Sci. USA, 99:3782, 2002; Wagner, D.H., Jr., et al., Int'lJ. of Mol. Med. 4:231, 1999), and are referred to as Th40 cells. (Waid, D.M., et al. (2004) Eur. J. of Immunol. 34:1488; Vaitaitis, G.M., et al., Cutting Edge, J. Immunol. 170:3455, 2003; Wagner, D.H., Jr., et al., Proc. Nat'l Acad. Sci. USA 99:3782, 2002; Wagner, D.H., Jr., et al., Int'l J. of Mol. Med. 4:231, 1999). CD40 expression typically is associated with antigen presenting cells and the majority of prior art describes CD40 as being expressed on B cells, macrophages, monocytes, and other cells; however, CD40 proteins are also expressed on T cells (Waid, D.M., et al., 2004. Eur. J. of Immunol., 34:1488, 2004; Vaitaitis, G.M., et al., Cutting Edge, J. Immunol., 170:3455, 2003; Wagner, D.H., Jr., et al., Proc. Nat'l Acad. Sci. USA, 99:3782, 2002; Wagner, D.H., et al., Int 'l. J. of Mol. Med., 4:231, 1999; Bourgeois, C., et al., Science, 297:2060, 2002; Fanslow, W.C., et al., J. of Immun., 152:4262, 1994; Ramsdell, F., et al., J. of Immunol. 152:2190, 1994; Grabstein, K.H., et al., J. of Immunol., 150:3141, 1993; Armitage, R.J., et al., Sem. in Immun., 5:401, 1993; Cooper, C.J., et al., J of Immunol., 173:6532, 2004). While Th40 cells comprise a proportion of the peripheral CD4+ compartment in naive, non-autoimmune mice (Waid, D.M., et al., Eur. J. of Immunol., 34:1488, 2004; Wagner, D.H., Jr., et al., Int'l J. of Mol. Med., 4:231, 1999), and in humans (Waid. D.M., et al., Clin. Immunol.. 124:138, 2007), this proportion is drastically expanded to as much as 50% of the CD4+ compartment in autoimmune prone mice (Waid, D.M., et al., Eur. J. of Immunol. 34:1488, 2004; Wagner, D.H., Jr., et al., Proc. Nat'l Acad. Sci. USA 99:3782, 2002; Wagner, D.H., et al., Int'l J. of Mol. Med., 4:231, 1999) and humans (Waid, D.M., et al., Eur. J. of Immunol. 34:1488, 2004; Waid. D.M., et al., Clin. Immunol. 124:138, 2007; Waid. D.M., et al., Clin. Immunol. 124:138, 2007). These T cells do not express early activation markers and occur in the naive phenotype of non-challenged mice.

In NOD (non-obese diabetic) mice, Th40 cells occur at exaggerated levels in spleen, lymph nodes and the pancreas, even prior to diabetes onset (Waid, D.M., et al., Eur. J. of Immunol. 34:1488, 2004; Wagner, D.H., Jr., et al., Proc. Nat'l Acad. Sci. USA 99:3782, 2002). An elevated number and percentage of these T cells are seen in peripheral blood of type 1 diabetic (T1D) patients when compared to non-autoimmune controls and type 2 diabetic patients (Waid. D.M., et al., Clin. Immunol., 124:138, 2007).

The observed increase in Th40 cells could mean that those T cells are antigen responsive or that CD40 expression is activation induced. Furthermore, several diabetogenic T cell clones are CD40+ (Wagner, D.H., Jr., et al., Proc. Nat'l Acad. Sci. USA 99:3782, 2002). Purified primary Th40 cells from NOD mice and from pre-diabetic NOD (12 - weeks of age) mice successfully transfer type 1 diabetes to NOD/scid (Non-Obese Diabetic/Severe Combined Immunodeficiency) recipient mice, directly demonstrating pathogenicity of the Th40 T cell subset (Waid, D.M., et al., Eur. J. of Immunol. 34:1488, 2004; Wagner, D.H., Jr., et al., 2002. Proc. Nat'l Acad. Sci. USA, 99:3782, 2002). It has been shown that Th40 cells infiltrate islet beta cells destroying insulin production thus suggesting islet antigen specificity (Waid, D.M., et al., Eur. J. of Immunol. 34:1488, 2004; Wagner, D.H., Jr., et al., Proc. Nat'l Acad. Sci. USA 99:3782, 2002). It has also been shown that Th40 cells are required for diabetes transfer. Peripheral (spleen and regional lymph node) T cells that were CD40 depleted, then CD25, Treg, depleted were not capable of transferring diabetes to Scid (Severe Combined Immunodeficiency) recipients. Even though Treg cells were removed, if the auto-aggressive CD40+ T cells subset is absent, disease transfer does not occur.

While Th40 cells are important in the development of autoimmunity, another important factor is expression of the CD40 - Ligand, CD154. CD154 is temporally induced on activated T-cells in response to CD3/TCR stimulation (Lederman, S. et al., J. of Exp. Med., 175:1091, 1992). CD154 expression has also been demonstrated on platelets, monocytes, basophils, eosinophils, dendritic cells, fibroblasts, smooth muscle, and endothelial cells (Russo, S. et al., J. Immunol. 171:5489, 2003; Stumpf, C., et al., Eur. J. Heart Fail., 5:629, 2003; Schonbeck, U., et al., Cell Mol. Life Sci. 58:4, 2001). CD154 is a member of the tumor necrosis factor (TNF) super-family and a soluble form of CD154 (sCD154) has been described (Russo, S., et al., J. Immunol. 171:5489 2003; Stumpf, C., et al., Eur. J. Heart Fail 5:629, 2003; Toubi, E., et al., Autoimmunity 37:457, 2004). Therefore, sCD154 may act like a cytokine (Stumpf, C., et al., Eur. J. Heart Fail. 5:629, 2003). Even though CD154 has not been genetically linked in T1D studies, sCD154 is significantly elevated in T1D and may play a role in the disease process (Varo, N. et al., Circulation 107:2664, 2003; Cipollone, F., et al., Diabetologia 48:1216, 2005; Devaraj, S., et al., Diabetes 55:774, 2006). The importance of CD40-CD154 interaction in autoimmunity has been established (Wagner, D.H., Jr., et al., Proc. Nat'l Acad. Sci. USA 99:3782, 2002; Kobata, T., et al., Rev. Immunogenet. 2:74, 2000; Homann, D., et al., Immunity 16:403, 2002; Goodnow, C. C., et al., Lancet 357:2115, 2001; Balasa, B., et al., J. of Immunol. 159:4620, 1997). Blocking CD40-CD154 interaction may prevent collagen induced arthritis, (Durie, F.H., et al., Science 281:1328, 1993) experimental autoimmune encephalitis (Howard, L.M., et al., Autoimmunity 37:411, 2004), prostatitis (Grossman, M.E., et al., J. Immunother. 24:237, 2001), and type-1 diabetes in the NOD mouse model (Durie, F.H. et al., Science 281:1328, 1993; Balasa, B. et al., Journal of Immunology 159:4620, 1997; Howard, L.M., et al., Autoimmunity 37:411, 2004; Grossman, M.E. et al., J. Immunother. 24:237, 2001). In the diabetes model, it was essential to administer a CD154 blocking antibody to NOD mice at 3-weeks of age because at 9-weeks, blocking antibodies had no effect on diabetes prevention (Balasa, B. et al., J. of Immunol. 159:4620, 1997).

Previous work has also demonstrated that the Th40 cell subset induces RAG1 and RAG2 (Recombination-Activating Genes) transcription, translation and nuclear translocation (Vaitaitis, G.M., et al., Cutting Edge, J. Immunol. 170:3455, 2003) when CD40 is engaged (Vaitaitis, G.M. et al., Cutting Edge, J. Immunol. 170:3455, 2003). CD3 engagement does not induce RAG1 or RAG2 in T-cells (Vaitaitis, G.M., et al., Cutting Edge, J. Immunol. 170:3455, 2003). Subsequent to RAG1/RAG2 induction, CD40-mediated T-cell receptor (TCR) revision occurs in peripheral T cells (Vaitaitis, G.M. et al., Cutting Edge, J. Immunol. 170:3455, 2003). CD40 induction of TCR revision is RAG dependent. T cells isolated from a TCR-Tg mouse undergo TCR revision when CD40 engaged, but T-cells from the TCR-Tg.RAG-/- mouse do not TCR revise when CD40 engaged (Wagner, D.H., Jr. et al., Int'l J. of Mol. Med. 4:231, 1999).

CD40 is a 50-kDa integral membrane protein of the tumor necrosis factor receptor (TNF-R) family. It is constitutively expressed as a homotrimer (Foy TM, et al., Ann. Rev. of Immunol., 14:591, 1996). In general, stimulation of all CD40-expressing cell types induces operations which contribute to inflammation, such as enhancement of costimulatory and adhesion molecules, and up-regulation of proteolytic enzymes (Mach, F. et al., Atherosclerosis. 137 Suppl: S89-95, 1998).

CD40's ligand - CD154 - is a 39-kDa protein that belongs to the tumor necrosis factor (TNF) family. CD40 forms a trimer that binds CD154 at the interface of the three monomers. CD154 is expressed commonly on cells beyond the surface-expressed CD154, as CD154 may also exist in a soluble biologically active form (sCD154) that is shed from the cell surface after activation. The main source of sCD154 is platelets. (Foy TM, et al., Ann. Rev. of Immunol., 14:591, 1996).

Genetically manipulated mouse models are utilized for research and development concerning atherosclerosis and cardiovascular disease because wild type mice are generally highly resistant to development and progression of atherosclerosis. Prior studies have attempted to block the CD40/CD154 interaction by using monoclonal antibodies and this approach has proven efficacious in several mouse model studies utilizing the Apoe-/- or LDLr deficient atherosclerotic models. Additionally, these same mouse models built with a deletion of CD154 saw significant reductions in overall plaque formation and may have also contributed to production of a more stable plaque phenotype. Clinically, stable plaques are identifiable and denoted by increased collagen and smooth muscle content, a thick fibrous cap, and an observable decrease in T cell, macrophage, and lipid accumulation.

Genetically manipulated mouse models are utilized for research and development concerning T2D because mouse models can portray insulin resistance and the inability of the beta cell to sufficiently compensate, which are characteristic of T2D in humans. Many animal models, including mouse models for T2D are obese, reflecting the human condition where obesity is closely linked to T2D development.

Multiple treatment options have been put forward to address and control both chronic and acute inflammation. Many approaches use non-steroidal anti-inflammatory drugs (NSAIDS) that attack the production of leukotrienes and prostaglandins, cellular products that cause localized inflammation. Other approaches use more powerful immunosuppressant drugs such as cyclophosphamide, methotrexate and azathioprine that suppress the immune response and stop the progression of the disease. Still other treatments involve the use of monoclonal antibodies (mAb) designed to alter the immune responses to self-tissues, as occurs during autoimmune diseases. However, all of these treatments often have severe, long-term side effects.

Current immune-modulatory therapies may rely upon monoclonal antibody treatments that may give rise to complications. For example, antibodies administered to a subject may cross-react with unintended targets and cause severe nephritic complications and those that specifically act against CD154 may cause embolic complications. Further, the CD40-CD154 interaction may play an important role in antibody generation which may indicate that administration of a monoclonal antibody could induce auto-antibody generation and further complications, which may inhibit the restoration of normal immune function (see generally Banchereau, J. et al., Annu. Rev. of Immunol. 12:881, 1994).

Other studies have demonstrated that blocking the CD154 interaction by using monoclonal antibodies, or limiting the CD40 receptor by monoclonal antibodies may abrogate atherosclerosis, and may confer a more favorable plaque phenotype characterized by lower inflammation and higher fibrosis. These studies additionally demonstrated that neointimal formation and restenosis may be limited by blocking the CD154 interaction. Studies concerning lupus nephritis may have demonstrated that blocking CD40 mediated signals can reduce anti-double-stranded DNA (anti-dsDNA) antibodies. Moreover, these studies may demonstrate that the reduction of anti-dsDNA was associated with increased serum complement levels and reduced glomerular inflammation, which may be viewed positively from a clinical perspective. However, the use of monoclonal antibodies to target the CD40/CD154 dyad was abandoned due to thromboembolic events which may have been related to the functioning of CD154 in thrombus stabilization. It is postulated that CD154 stabilize thrombi by interaction with the integrin α_{IIb}β₃, and by inhibiting CD154, thrombi may be less stable, and as a consequence shed emboli causing thrombotic events.

Studies of small molecules have also been conducted to attempt to inhibit the important CD40-CD154 costimulatory interaction required for T cell activation and the development of an effective immune response. For example, suramin, a symmetric polysulfonated napthylamine-benzamide urea derivative was studied for its ability to inhibit CD154 binding to its receptor and prevented the CD154-induced proliferation of human B cells; however, its numerous reversible toxicities (lethargy, rash, fatigue, anemia, hyperglycemia, hypocalcemia, coagulapathies, neutropenia, renal and hepatic complications) (Kaur, M. et al. (2002) Invest. New Drugs 20(2):209-19), loss of activity in protein-rich medium, and its interference with positive costimulatory interaction (Margolles-Clark, E. et al. (2009) Biochem. Pharmacol. 77(7):1236-45) made this and other related small-molecule candidates unlikely sources for effective therapy for the CD40-CD154 dyad.

Uncertainty exists regarding the primary lesion and the relative importance of the different tissues, metabolic defects in the liver and the peripheral tissues such as fat, muscle, and pancreatic β-cells likely all contribute to type 2 diabetes. Furthermore, although the cause of T2D is incompletely understood, complex and confounded by both genetic and environmental influences, hyperglycemia itself is believed to hinder pancreatic beta-cell function (Cernea, S., et al. Biochem. Med. (Zagreb) 2013;23(3):266-80).

Accordingly, type 2 diabetes in humans typically develops through a progressive series of increasingly disruptive phases or stages. Initially, pre-diabetes is characterized by impaired glucose tolerance, wherein the body has difficulty clearing glucose after a meal (postprandial hyperglycemia) and/or the body may have decreased sensitivity to insulin. In a second phase or stage, postprandial hyperglycemia and basal hyperglycemia occur while insulin producing beta cells of the pancreas become dysfunctional at an increasing rate. During the next phase of the disease progression, hyperglycemia occurs even after fasting and at a cellular level significant beta cell atrophy takes place. Ultimately, in the final phase or end stage of disease progression, beta cells can no longer produce and/or release insulin and the patient requires insulin replacement therapy.

T2D is clinically characterized by hyperglycemia and pathologically by insulin resistance with relative insulin secretory impairment. Individuals who are genetically prone to developing T2D may experience insulin resistance (the earliest detectable metabolic defect) between 15 and 25 years or more before the clinical onset of overt diabetes. (Kahn, CR, Diabetes. (1994) 43:1066-1084). T2D classically has been associated with age and obesity; however, the increased diagnosis of youth with T2D has demonstrated that these two factors alone are not the sole reliable predictors of the disease. Body mass index (BMI) is also clearly associated with T2D, but both genetic and environmental factors are now identified as contributory as well (Wu Y, et al. Int. J. Med. Sci. (2014) 11(11):1185-200. Epub 2014/09/06; Cefalu, WT, Diabetes. (2009) 58(2):307-8; Donath, MY, et al., Nat. Rev. Immunol. (2011) 11(2):98-107). Obesity creates inflammatory conditions, and sustained inflammation resulting from obesity or other conditions may be important in T2D development (Donath, MY, et al., Nat. Rev. Immunol. (2011) 11(2):98-107; Donath, MY, Nat. Rev. Drug Discov. (2014) 13(6):465-76).

Thus, there exists a need in the art for safer and more effective methods for treatment and prevention of T2D implicated by the autoimmune and inflammatory pathway and dyad related to CD40-CD154. The present developments may address this need by describing methods for treatment of T2D by administration of a therapeutically effective amount of CD40-binding peptide.

Thus, there exists a need in the art for safer and more effective methods for treatment and prevention of cardiovascular diseases (CVDs) and T2D implicated by aseptic chronic inflammation (ACI). The present developments may address this need by describing methods for treatment of atherosclerosis by administration of a therapeutically effective amount of a peptide that affects, regulates, blocks, inhibits, or modulates the CD40-CD154 dyad. Further, the present developments may provide the added benefit of preventing auto-antibody generation, and thus allow the resumption of normal immune function.

This statement of background is for information purposes only and is not intended to be a complete or exhaustive explication of all potentially relevant background.

### Summary

The present developments provide a peptide that comprises an amino acid sequence of SEQ ID NO: 3 for use in a method for preventing, reducing, treating and/or reversing T2D in a subject, the method comprising administering to the subject an effective amount of the peptide, wherein the peptide is no more than 25 amino acids in length and that affects the interaction of CD40 with CD154/CD40-ligand by inhibiting the binding of CD40 to CD154..

The present developments are based on the knowledge that interaction of CD40-ligand (CD154 protein) with CD40 protein expressed on T-cells (Th40 cells), may be important in the development of type 2 diabetes and autoimmune diseases. The present developments may be based on the elucidation of the critical residues in CD40 and CD154 that may be important for this interaction. The present developments relate to small synthesized peptides for use for blocking and/or disrupting the interaction between a CD40 protein and a CD154 protein wherein the peptides interact and/or associate with the CD40 protein at a site where the CD154 protein would normally bind. The present developments also relate to such peptides for use to reduce the level of Th40 cells, thereby reducing the severity of disease. The peptides for use of the current developments may bind directly and/or alternatively associate with the CD40 molecule in such a way as to alter CD40 function.

In autoimmune diseases and conditions, CD40 engagement may promote inflammation. Accordingly, in one embodiment of the present developments the peptide for use may alter CD40 signals to no longer be inflammatory. Thus, in one embodiment the peptides for use of the current development may block, disrupt, interfere, and/or inhibit CD40 function at sites including but not limited to Th40 cells, pancreas beta cells, endothelial cells, B cells, monocytes, and/or macrophages. The current developments contemplate the small interfering peptides (SIPs) for use disclosed herein to interfere with the CD40-signaling pathway of any cells that may present with CD40. These peptides may be able to interfere at specific sites depending on the route of administration.

One embodiment of the present developments is addressed to the peptide for use in a method for preventing, reducing, and/or reversing type 2 diabetes, the method comprising contacting the CD40 protein with the peptide that interacts with the CD40 protein. Said peptides are those that are less than 25 amino acids in length, and that bind to a CD40 protein, thereby inhibiting its interaction with a CD154 protein.

One embodiment of the present developments is addressed to the peptide for use in a method for preventing, reducing and/or reversing type 2 diabetes, the method comprising inhibiting interaction between a CD40 protein and a CD154 protein with the peptide that interacts with the CD40 protein. Preferred peptides for use interact with the CD40 protein at the CD154 - binding site. Such peptides are less than 25 amino acids in length. Preferred peptides for use are those amino acid sequences selected from SEQ ID NOs 3-9 and 25-30.

One embodiment of the present developments is addressed to the peptide for use in a method to prevent, reduce and/or reverse type 2 diabetes in an animal, the method comprising administering to the animal, the peptide that interacts with a CD40 protein in such a manner as to modulate glucose transport protein 4 ("GLUT4"). Preferred peptides for use are those that interact with the CD40 protein at the CD154 -binding site, thereby modulating GLUT4. Preferred peptides for use may modulate, upregulate, or increase GLUT4 in both adipose and muscle tissue compared to untreated populations. Such peptides are for use to prevent and/or reduce T2D and symptoms that may accompany T2D.

Another embodiment of the present developments, is addressed to the peptide for use in a method to administer a CD40-binding peptide to prevent, reduce and/or reverse type 2 diabetes, wherein the peptide interacts with a CD40 protein and CD154 binding site, and is administered using a delivery method selected from intramuscular (IM) delivery, intravenous (IV) delivery, subcutaneous (SC) delivery, oral delivery, gavage delivery, emollient/skin delivery, transdermal patch, or nasal administration.

Another embodiment of the present developments, is addressed to the peptide for use in a method to prevent, reduce and/or reverse type 2 diabetes in an animal, wherein the peptide interacts with a CD40 protein and CD154 binding site, and is administered using an extended delivery method selected from an implantable device, a hydrophilic polymer formulation, a permeable polymeric membrane, injectable gel implants, solvent extraction system, phase inversion system, thermosensitive gels, pH dependent *in situ* gels, microparticles, microspheres, nanoparticles, nanospheres, bio-degradable implants, or photoactivated depot.

Another embodiment of the present developments, is addressed to the peptide for use, selected from SEQ ID NOs 3-9 and 25-30, wherein the peptide is administered in an amount sufficient to reduce or inhibit interleukin-2 signaling, wherein, the interleukin-2 signaling is associated with type 2 diabetes in the subject.

Another embodiment of the present developments is addressed to the peptide for use, selected from SEQ ID NOs 3-9 and 25-30, wherein the peptide is administered in an amount sufficient to reduce or inhibit IFN-γ signaling, wherein, the IFN-y signaling is associated with type 2 diabetes in the subject.

Another embodiment of the present developments, is addressed to the peptide for use, selected from SEQ ID NOs 3-9 and 25-30, wherein the peptide is administered in an amount sufficient to change interleukin-21 (IL-21), interleukin-22 (IL-22), IFNγ, TNFα, interleukin-6 (IL-6), granulocyte-macrophage colongy-stimulating factor (GM-CSF), interleukin-4 (IL-4), interleukin-10 (IL-10) and transforming growth factor beta (TGFβ), wherein the said interleukin-21 (IL-21), interleukin-22 (IL-22), IFNγ, TNFα, interleukin-6 (IL-6), granulocyte-macrophage colongy-stimulating factor (GM-CSF), interleukin-4 (IL-4), interleukin-10 (IL-10) and transforming growth factor beta (TGFβ) signaling is associated with type 2 diabetes in the subject.

Another embodiment of the present developments includes the peptide for use, selected from SEQ ID NOs 3-9 and 25-30, wherein the peptide is administered in an amount sufficient to reduce or inhibit interleukin 17 (IL-17) signaling, wherein, the IL-17 is associated with type 2 diabetes in the subject.

### Brief Description of the Figures

Fig. 1 is a chart of the effect of various peptides of CD154 on the development of diabetes in NOD mice. The 8-mer (SEQ ID NO: 5), 10-mer (SEQ NO: 24), 13-mer (SEQ ID NO:25), 15-mer (SEQ ID NO: 7), and 24-mer (SEQ ID NO:26) were tested.
Fig. 2A is a chart of the effect of a 15-mer peptide from CD154 on the CD4/CD8 ratio in NOD mice.
Fig. 2B is a chart of the effect of 15-mer peptide on beta-islet infiltration in treated versus control pancreata excised, examined, and scored.
Fig. 3 is a graph of reversal of diabetes in NOD mice using a 15-mer peptide from CD154.
Fig. 4 is a dot-plot of the detection of Th40 cells using a SIP-15-mer peptide from CD154.
Fig. 5 is a dot-plot of a screening of B cells using a SIP-15-mer peptide from CD154.
Fig. 6 is a chart demonstrating a comparison of Th40 cell levels in diabetic and non-diabetic mice.
Fig. 7 is a chart demonstrating the effect of treatment with the 15-mer peptide on insulin granulation of the pancreas.
Fig. 8 is a graph that shows the effect of mutations in the 15-mer peptide on the ability of the 15-mer peptide to inhibit development of diabetes in NOD mice.
Fig. 9 is a chart showing the number of cells (x10⁶) of CD3+CD4+CD40+ in different mice models.
Fig. 10 is a chart showing the percentage of Th40 cells in the peripheral blood in human subjects in control and diabetic subjects.
Fig. 11 is a dot-plot comparing CD4+ and CD40 cell data obtained through flow cytometry. Th40 cells (CD4+CD40+) are in the upper right quadrant.
Fig. 12 is a chart showing the percentage of Th40 cells of CD3+CD4+ population of mice models.
Fig. 13 is a chart that shows Th40 Cell percentage of CD3+CD4+ population within the murine aorta of C57B-6, young non-diabetic NOD mice, and diabetic NOD mice.
Fig. 14 is an image at 200x magnification showing Th40 cells in the shoulder region of plaque in the ApoE-/- mouse model.

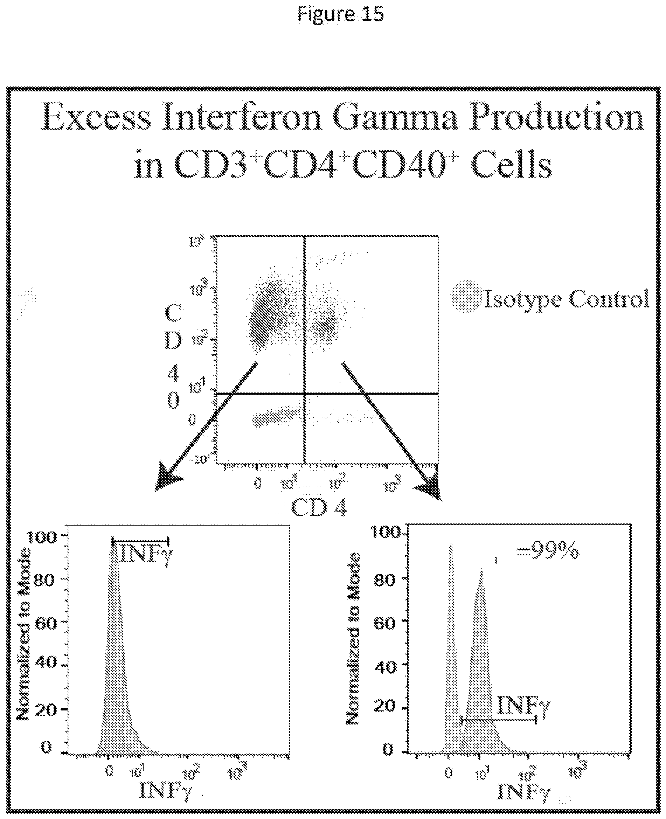

Fig. 15 is a graph of interferon gamma control of Th40 proliferation.
Fig. 16 is a plot of CD40 stimulated proliferation of Th40 cells in the absence/presence of anti-interferon gamma antibody (αIFNγ) demonstrating that interferon gamma mediates CD40 induced proliferation.
Fig. 17 is a sample of stains of aortic arch of the lesser curvature of the aortic arch.
Fig. 18 is a stain of lesser curvature of aortic arch in control and treated subjects.
Fig. 19 is a chart of area measurements of the lesser curvature of aortic arches and plaques.
Fig. 20-23 are charts of treated and control ApoE mice subjects.
Fig. 24 is a table of blood clot data from human blood in the presence of 15-mer peptide compared to normal clotting.

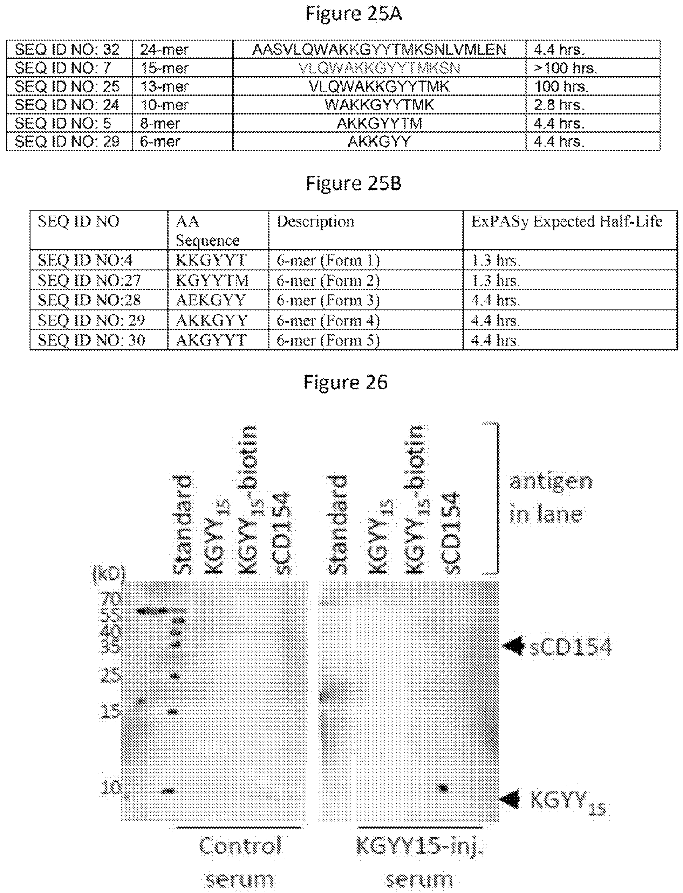

Fig. 25A is a table providing the relative peptide stability assessed by ExPASy analysis.
Fig. 25B is a table providing the relative peptides stability assessed by ExPASy analysis.
Fig. 26 is a western blot comparing control and treated samples from subject mice.
Fig. 27 is a graph of LDL cholesterol measured in treated and untreated subjects.
Fig. 28A is an image of KGYY6 treated aortic en-face Sudan IV staining.
Fig. 28B is an image of control aortic en-face Sudan IV staining.
Fig. 29 is a graph demonstrating the reduction of lesion areas of Sudan IV staining.
Fig. 30 is a graph of plaque volume reduction for area under the curve.
Fig. 31 is a graph of plaque composition for KGYY6 (SEQ ID NO:29) treated and control subject mice.
Fig. 32A is an image of trichrome stained sections of KGYY6 (SEQ ID NO:29) treated subject.
Fig. 32B is an image of trichrome stained sections of control subjects.
Fig. 33(a) - (b) are graphs that show a statistically significant improvement in glucose tolerance (GTT) and insulin sensitivity in response to SEQ ID NO: 29.
Fig. 34 is a western blot analysis of GLUT4 (insulin regulated glucose transport protein) comparing adipose and muscle tissue of treated (with SEQ ID NO: 29) and untreated mice.
Fig. 35 is a graph of the percentage change of in-vitro lymphocyte cytokines measured in spleen cells from ApoE-/- and C57BL/6 mice as measured by flow cytometry.

### Detailed Description

The present subject matter is based on the discovery that a unique subset of T-cells, which express CD40 protein, and thus are referred to as Th40 cells, may be instrumental in autoimmune inflammation. Moreover, involvement of Th40 cells in the autoimmune process may be dependent on the interaction between CD40 protein expressed on the surface of the T-cell, and CD154 protein. Interaction of CD40 and CD154 results in activation signals being delivered between the cells, and subsequent activation of the Th40 cell. Such activation results in propagation of the Th40 cell and an increase in inflammation (e.g., an increase in the number of immune cells and immunoregulatory molecules, present in the system). Accordingly, inhibition of the CD40/CD154 interaction can modulate Th40 cell activity, and thereby affect inflammation. Thus the present subject matter relates to the peptides, and administration thereof, that may affect the interaction between a CD40 protein and a CD154 protein, thereby modulating inflammation. Moreover, the present subject matter relates to peptides that affect the interaction between CD40 protein expressed on the surface of a T-cell, and a CD154 protein, thereby affecting T-cell activity, controlling inflammation, and consequently preventing, modulating, and reducing atherosclerosis.

Before the present development is further described, it is to be understood that this invention is not strictly limited to particular embodiments described, as such may of course vary. It is also to be understood that the terminology used herein is for the purpose of describing particular embodiments only, and is not intended to be limiting, since the scope of the present invention will be limited only by the claims.

It must be noted that as used herein and in the appended claims, the singular forms "a," "an," and "the" include plural referents unless the context clearly dictates otherwise. It should further be understood that as used herein, the term "a" entity or "an" entity refers to one or more of that entity. For example, a nucleic acid molecule refers to one or more nucleic acid molecules. As such, the terms "a", "an", "one or more" and "at least one" can be used interchangeably. Similarly, the terms "comprising", "including" and "having" can be used interchangeably.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments are specifically embraced by the present invention and are disclosed herein just as if each and every combination was individually and explicitly disclosed. In addition, all sub-combinations are also specifically embraced by the present invention and are disclosed herein just as if each and every such sub-combination was individually and explicitly disclosed herein.

It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Furthermore, as used herein the term animal refers to a vertebrate, preferably a mammal, more preferably a human. Suitable mammals on which to use the peptides for use of the present invention include but are not limited farm animals, sports animals, pets, primates, mice, rats, horses, dogs, cats, and humans. The term animal can be used interchangeably with the terms subject or patient.

. As used herein, the terms interact, interaction, and the like, mean that two molecules come into sufficient physical proximity such that they cause a modulation of inflammation. One such type of interaction is a binding interaction. In such an interaction the peptide associates with CD40 to form a complex. An example of complex formation is the association of an antigen with an antibody. According to the present subject matter, binding of a peptide hereof to a CD40 protein can be reversible (e.g., non-covalent binding interactions) or non-reversible (e.g., covalent binding interactions). Moreover, a reversible interaction can be strong or weak, the strength of the interaction being determined by the forces (e.g., ionic charges, hydrogen binding, van der Walls interactions, etc.) exerted by each protein on the other protein in the complex. Factors affecting the strength of an interaction between two molecules are known to those skilled in the art. One useful measure of the strength of binding between two molecules, such as a peptide and a protein, is the dissociation constant (Kd). Preferred peptides for use of the present invention are those that bind to a CD40 protein with a Kd of no more than about 1 x 10⁻⁶ M, about 1 x 10⁻⁷ M, or about 1 x 10⁻⁸ M. Particularly preferred peptides for use are those having a Kd of less than about 1 x 10⁻⁹ M. In one embodiment, a peptide for use hereof binds to a CD40 protein with a Kd of less than 100 nM, less than 50 nM, less than 25 nM, less than 10 nM, less than 5 nM, less than 3 nM, less than 2 nM, or less than 1 nM. Methods of measuring and analyzing binding interactions between a peptide and a CD40 protein are known by those of skill in the art.

As used herein, the change the level of Th40 cells present in an animal, or in a culture of T cells may be indicative of modulation of inflammation. As used herein, the terms level, number, count and concentration can be used interchangeably. Modulation of inflammation may mean an increase or decrease in the number of Th40 cells present in the inflammatory environment; however, the modulation of inflammation should not be limited to cell numbers or counts. Consequently, modulation can be referred to as positive or negative. Positive modulation (also referred to as up-regulation) of inflammation may result in an increase in the number of Th40 cells in the inflammatory environment. Negative modulation (also referred to as down-regulation) of inflammation may result in a reduction in the number of Th40 cells present in the inflammatory environment. The level, count, or concentration of Th40 cells may not be indicative of inflammation in the inflammatory environment. A preferred peptide for use is one that down-regulates inflammation, thereby reducing the number of Th40 cells present in the inflammatory environment. Positive and negative modulation of inflammation may or may not result in a change in the type and amount of immunoregulatory molecules present in the inflammatory environment. In some instances, it is possible that the Th40 levels will not change but the activity of those cells is altered such that they are no longer exerting or increasing inflammatory cytokines and other biomarkers of inflammation. Therefore, as used herein modulating inflammation may refer to changes in the Th40 levels, numbers, or concentration in an corporeal body or sample, and may also refer to changes in inflammation more generally that may be associated with disease, inflammatory cytokines, and/or cell derived inflammatory mediator molecules.

It will be appreciated by those skilled in the art that both a cell culture system and the immune system of an animal comprise basal levels of immune cells and immunoregulatory molecules. The phrases basal level and normal level can be used interchangeably. With regard to the immune system of an animal, as used herein, the basal level of a type of immune cell (e.g., Th40 cell), or a immunoregulatory molecule, refers to the average number of that cell type, or immunoregulatory molecule, present in a population of individuals considered healthy (i.e., free of metabolic, autoimmune, or infectious disease). With regard to a cell culture system, as used herein, the basal level of a type of immune cell, or an immunoregulatory molecule, refers to the average level of that cell type, or immunoregulatory molecule, present in a population of cells that is non-activated. Those skilled in the art are capable of determining if a T-cell, or a population of such cells, is activated. For example, the expression of CD69, CD25 and/or CD154 proteins by a cell indicates that the cell has been activated.

The basal level of a cell or molecule can be a specific amount (e.g., a specific concentration) or it can encompass a range of amounts. Basal levels, or ranges, of immune cells and immunoregulatory molecules are known to those in the art. For example, in a healthy individual, the normal level of CD4+ T-cells present in human blood is 500-1500 cells/ml. Variability in this measurement can result from differences in the method used to determine the cell count. Furthermore, normal levels of cells can also be reported as a percentage of a total cell population. For example, in a healthy individual, Th40 cells make up less than 25% of the total T cell population. Thus, as used herein, the term inflammation refers to an inflammatory environment in which Th40 cells make up greater than about 25%, greater than about 30%, greater than about 35%, greater than about 40%, greater than about 45% , greater than about 50%, greater than about 55%, greater than about 60%, greater than about 65%, greater than about 70%, greater than about 75%, or greater than about 80% of the total T-cell population. Moreover, a preferred peptide for use herein is one that reduces the level of Th40 cells to less than about 50%, less than about 45%, less than about 40%, less than about 35%, less than about 30%, less than about 27%, or equal to about 25% of the total T-cell population. Methods of measuring different types of T-cells in the T-cell population are known to those skilled in the art. Furthermore, a novel method for detecting Th40 cells using peptides hereof is disclosed herein.

As used herein, the phrase inflammatory environment refers to the overall population of immune cells, and related immunoregulatory molecules, that are present in a culture of cells, or in the body of an animal. As such, the phrase inflammatory environment encompasses the types, and/or the relative amounts of immune cells and immunoregulatory molecules (e.g., cytokines) present in a culture of cells, or in an animal, which are involved in affecting an inflammatory reaction. Examples of cells encompassed by the term inflammatory environment include, but are not limited to, T cells, neutrophils, macrophages, granulocytes, and the like. The inflammatory environment relates to cells and molecules that mediate both acute and chronic inflammation. It will be appreciated by those skilled in the art that the inflammatory environment refers to the system to which peptides hereof are administered. In one embodiment, the system is a cell culture system. In one embodiment, the system is a whole animal.

A preferred peptide for use hereof is one that selectively interacts with a CD40 protein in solution, as determined using an assay such as an immunosorbent assay, or on the surface of a T-cell. As used herein, the terms selectively, selective, specific, and the like, indicate the peptide has a greater affinity for a CD40 protein than it does for proteins unrelated to the CD40 protein. More specifically, the terms selectively, selective, specific, and the like indicate that the affinity of the peptide for CD40 is statistically significantly higher than its affinity for a negative control (e.g., an unrelated protein such as albumin) as measured using a standard assay (e.g., ELISA). Suitable techniques for assaying the ability of a peptide to selectively interact with a CD40 protein are known to those skilled in the art. Such assays can be in vitro or in vivo assays. Examples of useful assays include, but are not limited to, an enzyme-linked immunoassay, a competitive enzyme-linked immunoassay, a radioimmunoassay, a fluorescence immunoassay, a chemiluminescent assay, a lateral flow assay, a flow-through assay, an agglutination assay, a particulate-based assay (e.g., using particulates such as, but not limited to, magnetic particles or plastic polymers, such as latex or polystyrene beads), an immunoprecipitation assay, an immunoblot assay (e.g., a western blot), a phosphorescence assay, a flow-through assay, a chromatography assay, a polyacrylamide gel electrophoresis (PAGE)-based assay, a surface plasmon resonance assay, a spectrophotometric assay, a particulate-based assay, an electronic sensory assay and a flow cytometric assay. Methods of performing such assays are well known to those skilled in the art. In one embodiment, an assay can be performed using cells in culture, or it can be performed in a whole animal. Assays can be designed to give qualitative, quantitative or semi-quantitative results, depending on how they are used and the type of result that is desired.

### Type 2 Diabetes Related Developments

The present subject matter is based on the discovery that a unique subset of T-cells, which express CD40 protein, and thus are referred to as Th40 cells, that may be instrumental in autoimmune inflammation, including conditions such as type 2 diabetes. Moreover, involvement of Th40 cells in the autoimmune process may be dependent on the interaction between CD40 protein expressed on the surface of the T-cell, and CD154 protein. Interaction of CD40 and CD154 results in activation signals being delivered between the cells, and subsequent activation of the Th40 cell. Such activation results in propagation of the Th40 cell and an increase in inflammation (e.g., an increase in the number of immune cells and immunoregulatory molecules, present in the system). Accordingly, inhibition of the CD40/CD154 interaction can modulate Th40 cell activity, and thereby affect inflammation. Thus the present subject matter relates to the peptides, and administration thereof, that may affect the interaction between a CD40 protein and a CD154 protein, thereby modulating inflammation. Moreover, the present subject matter relates to peptides that affect the interaction between CD40 protein expressed on the surface of a T-cell, and a CD154 protein, thereby affecting T-cell activity, controlling inflammation, and consequently preventing, modulating, reducing and/or reversing type 2 diabetes.

One embodiment of the present subject matter is addressed to the peptide for use, wherein the peptide interacts with a CD40 protein in such a manner as to prevent type 2 diabetes. As used herein, the terms interact, interaction, and the like, mean that two molecules come into sufficient physical proximity such that they cause a modulation of inflammation. One such type of interaction is a binding interaction. In such an interaction the peptide associates with CD40 to form a complex. An example of complex formation is the association of an antigen with an antibody. According to the present subject matter, binding of a peptide hereof to a CD40 protein can be reversible (e.g., non-covalent binding interactions) or non-reversible (e.g., covalent binding interactions). Moreover, a reversible interaction can be strong or weak, the strength of the interaction being determined by the forces (e.g., ionic charges, hydrogen binding, van der Walls interactions, etc.) exerted by each protein on the other protein in the complex. Factors affecting the strength of an interaction between two molecules are known to those skilled in the art. One useful measure of the strength of binding between two molecules, such as a peptide and a protein, is the dissociation constant (Kd). Preferred peptides for use of the present invention are those that bind to a CD40 protein with a Kd of no more than about 1 x 10⁻⁶ M, about 1 x 10⁻⁷ M, or about 1 x 10⁻⁸ M. Particularly preferred peptides for use are those having a Kd of less than about 1 x 10⁻⁹ M. In one embodiment, a peptide for use hereof binds to a CD40 protein with a Kd of less than 100 nM, less than 50 nM, less than 25 nM, less than 10 nM, less than 5 nM, less than 3 nM, less than 2 nM, or less than 1 nM. Methods of measuring and analyzing binding interactions between a peptide and a CD40 protein are known by those of skill in the art.

As used herein, the change the level of Th40 cells present in an animal, or in a culture of T cells may be indicative of modulation of inflammation. As used herein, the terms level, number, count and concentration can be used interchangeably. Modulation of inflammation may mean an increase or decrease in the number of Th40 cells present in the inflammatory environment; however, the modulation of inflammation should not be limited to cell numbers or counts. Consequently, modulation can be referred to as positive or negative. Positive modulation (also referred to as up-regulation) of inflammation may result in an increase in the number of Th40 cells in the inflammatory environment. Negative modulation (also referred to as down-regulation) of inflammation may result in a reduction in the number of Th40 cells present in the inflammatory environment. The level, count, or concentration of Th40 cells may not be indicative of inflammation in the inflammatory environment. A preferred peptide for use may be one that down-regulates inflammation, thereby reducing the number of Th40 cells present in the inflammatory environment. Positive and negative modulation of inflammation may or may not result in a change in the type and amount of immunoregulatory molecules present in the inflammatory environment. In some instances, it is possible that the Th40 levels will not change but the activity of those cells is altered such that they are no longer exerting or increasing inflammatory cytokines and other biomarkers of inflammation. Therefore, as used herein modulating inflammation may refer to changes in the Th40 levels, numbers, or concentration in a corporeal body or sample, and may also refer to changes in inflammation more generally that may be associated with disease, inflammatory cytokines, and/or cell derived inflammatory mediator molecules.

It will be appreciated by those skilled in the art that both a cell culture system and the immune system of an animal comprise basal levels of immune cells and immunoregulatory molecules. The phrases basal level and normal level can be used interchangeably. With regard to the immune system of an animal, as used herein, the basal level of a type of immune cell (e.g., Th40 cell), or a immunoregulatory molecule, refers to the average number of that cell type, or immunoregulatory molecule, present in a population of individuals considered healthy (i.e., free of metabolic, autoimmune, or infectious disease). With regard to a cell culture system, as used herein, the basal level of a type of immune cell, or an immunoregulatory molecule, refers to the average level of that cell type, or immunoregulatory molecule, present in a population of cells that is non-activated. Those skilled in the art are capable of determining if a T-cell, or a population of such cells, is activated. For example, the expression of CD69, CD25 and/or CD154 proteins by a cell indicates that the cell has been activated.

The basal level of a cell or molecule can be a specific amount (e.g., a specific concentration) or it can encompass a range of amounts. Basal levels, or ranges, of immune cells and immunoregulatory molecules are known to those in the art. For example, in a healthy individual, the normal level of CD4+ T-cells present in human blood is 500-1500 cells/ml. Variability in this measurement can result from differences in the method used to determine the cell count. Furthermore, normal levels of cells can also be reported as a percentage of a total cell population. For example, in a healthy individual, Th40 cells make up less than 25% of the total T cell population. Thus, as used herein, the term inflammation refers to an inflammatory environment in which Th40 cells make up greater than about 25%, greater than about 30%, greater than about 35%, greater than about 40%, greater than about 45% , greater than about 50%, greater than about 55%, greater than about 60%, greater than about 65%, greater than about 70%, greater than about 75%, or greater than about 80% of the total T-cell population. Moreover, a preferred peptide for use herein is one that reduces the level of Th40 cells to less than about 50%, less than about 45%, less than about 40%, less than about 35%, less than about 30%, less than about 27%, or equal to about 25% of the total T-cell population. Methods of measuring different types of T-cells in the T-cell population are known to those skilled in the art. Furthermore, a novel method for detecting Th40 cells using peptides hereof is disclosed herein.

As used herein, the phrase inflammatory environment refers to the overall population of immune cells, and related immunoregulatory molecules, that are present in a culture of cells, or in the body of an animal. As such, the phrase inflammatory environment encompasses the types, and/or the relative amounts of immune cells and immunoregulatory molecules (e.g., cytokines) present in a culture of cells, or in an animal, which are involved in affecting an inflammatory reaction. Examples of cells encompassed by the term inflammatory environment include, but are not limited to, T cells, neutrophils, macrophages, granulocytes, and the like. The inflammatory environment relates to cells and molecules that mediate both acute and chronic inflammation. It will be appreciated by those skilled in the art that the inflammatory environment refers to the system to which peptides hereof are administered. In one embodiment, the system is a cell culture system. In one embodiment, the system is a whole animal.

A preferred peptide for use hereof is one that selectively interacts with a CD40 protein in solution, as determined using an assay such as an immunosorbent assay, or on the surface of a T-cell. As used herein, the terms selectively, selective, specific, and the like, indicate the peptide has a greater affinity for a CD40 protein than it does for proteins unrelated to the CD40 protein. More specifically, the terms selectively, selective, specific, and the like indicate that the affinity of the peptide for CD40 is statistically significantly higher than its affinity for a negative control (e.g., an unrelated protein such as albumin) as measured using a standard assay (e.g., ELISA). Suitable techniques for assaying the ability of a peptide to selectively interact with a CD40 protein are known to those skilled in the art. Such assays can be in vitro or in vivo assays. Examples of useful assays include, but are not limited to, an enzyme-linked immunoassay, a competitive enzyme-linked immunoassay, a radioimmunoassay, a fluorescence immunoassay, a chemiluminescent assay, a lateral flow assay, a flow-through assay, an agglutination assay, a particulate-based assay (e.g., using particulates such as, but not limited to, magnetic particles or plastic polymers, such as latex or polystyrene beads), an immunoprecipitation assay, an immunoblot assay (e.g., a western blot), a phosphorescence assay, a flow-through assay, a chromatography assay, a polyacrylamide gel electrophoresis (PAGE)-based assay, a surface plasmon resonance assay, a spectrophotometric assay, a particulate-based assay, an electronic sensory assay and a flow cytometric assay. Methods of performing such assays are well known to those skilled in the art. In one embodiment, an assay can be performed using cells in culture, or it can be performed in a whole animal. Assays can be designed to give qualitative, quantitative or semi-quantitative results, depending on how they are used and the type of result that is desired.

One embodiment hereof is a peptide for use that interacts with a CD40 protein in such a manner as to affect the interaction of the CD40 protein with a CD154 protein, thereby modulating inflammation. The effect of the peptide on the CD40/CD154 interaction can be positive or it can be negative. For example, the peptide can interact with the CD40 protein in such a manner that the strength of the interaction between the CD40 protein and a CD154 protein is increased. Alternatively, the peptide can interact with the CD40 protein such that the strength of the interaction between the CD40 protein and a CD154 protein is decreased. Methods of measuring the strength of binding between the peptide and a CD40 protein are known to those skilled in the art. The peptide for use hereof is one that reduces the strength of the interaction between a CD40 protein and a CD154 protein. Preferred peptides for use hereof reduce the strength of binding between a CD40 protein and a CD154 protein by at least 10%, at least 20%, at least 30%, at least 40%, at least 50%, at least 60%, at least 70%, at least 80%, at least 90%, or at least 95%. A particularly preferred peptide for use is one that completely inhibits binding of CD40 to CD154. Complete inhibition of binding between CD40 and CD154 means that when a peptide hereof is brought into proximity with a CD40 protein and a CD154 protein under conditions that would normally allow the interaction of CD40 and CD154, no such interaction occurs and activation signals are not stimulated in the CD40-expressing cell. Consequently CD40/CD154 mediated modulation of inflammation does not occur. In one embodiment, the peptide for use interacts with the CD40 protein in such a manner as to reduce the level of inflammation in the system. In one embodiment, the peptide for use interacts with the CD40 protein in such a manner as to inhibit the development of inflammation in the system. In one embodiment, the peptide for use may alter the way the cells may interact with other molecules that normally occur during cell-to-cell interactions. Such cell-to-cell interactions may be those that result in inflammation. The peptides for use of the developments hereof, may disrupt the inflammasome complex, which may promote the maturation of pro-inflammatory cytokines interleukin-1β(IL-1β) and interleukin-18 (IL-18). Furthermore, one embodiment of the peptides for use hereof is that these peptides may disrupt the inflammasome complex of caspase 1, PYCARD, NALP, caspase 5, nucleotide-binding oligomerization domain and leucine-rich repeat-containing receptors (NLRs) and ALRs (AIM2-like receptors).

One embodiment of the peptides for use described herein is that administration of the peptide may alter the way the cell may interact with other molecules that normally occur during cell to cell interactions which may be indicative of or result in inflammation. One embodiment of the peptides for use described herein is that these peptide(s) may disrupt the inflammasome and thus alter inflammatory outcomes.

While peptides for use hereof can interact with any site on the CD40 protein, preferred peptides for use interact with the CD40 protein at a location that overlaps with the CD154 binding site. In one embodiment, a peptide for use hereof interacts with the CD40 protein at the CD154 binding site. An example of such a peptide is a CD40 ligand competitive antagonist. As used herein, peptides that interfere with, or inhibit, the binding of a CD154 protein to a CD40 protein are referred to as small interfering peptides (SIPs). As used herein a small interfering peptide is a peptide that, through physio-chemical properties, interferes with the interaction of a CD40 protein with a CD154 protein, thereby preventing activation signals from being delivered to the CD40-bearing cell, thus limiting the activation of the CD40-bearing cell, and consequently, inflammation. As demonstrated herein, the consequences of such interference are prevention of T-cell activation and propagation, and a prevention or reduction of inflammation.

Additionally, a small interfering peptide, may, through its physio-chemical properties, interfere with the interaction of a CD40 protein with a CD154 protein, thereby preventing activation signals from being delivered to the CD40-bearing cell, thus limiting the activation of the CD40-bearing cell, and consequently, modulating, inhibiting, preventing, and/or reversing type 2 diabetes. As demonstrated herein, the consequences of such interference are prevention of T cell activation and propagation, and a prevention, reduction, modulation, and reversal of type 2 diabetic developments.

A peptide useful for use in practicing methods of the present developments should be of a size sufficient to interact with CD40 protein in such a manner as to modulate type 2 diabetes. It is understood by those skilled in the art that preferred peptides for use are relatively short since they are easier and less expensive to produce. Preferred peptides for use are those that are less than 20 amino acids in length. A preferred peptide for use is one that is 4, 6, 8, 10, 13, 15, or 24 amino acids in length. In one embodiment, the peptide for use is an amino acid selected from the group of SEQ ID NO:3 (Core-sequence see Table 1), SEQ ID NO:4 (6-mer see Table 1), SEQ ID NO:5 (8-mer mouse see Table 1), SEQ ID NO:6 (8-mer human see Table 1), SEQ ID NO:7 (15-mer mouse see Table 1), SEQ ID NO:8 (15-mer human see Table 1), SEQ ID NO:9 (24-mer see Table 1), SEQ ID NO: 24 (10-mer see Table 1), SEQ ID NO: 25 (13-mer see Table 1), SEQ ID NO: 26 (24-mer see Table 1), SEQ ID NO: 27 (6-mer (Form 2) see Table 1), SEQ ID NO: 28 (6-mer (Form 3) see Table 1), SEQ ID NO: 29 (6-mer (Form 4) see Table 1), SEQ ID NO: 30 (6-mer (Form 4) see Table 1) and SEQ ID NO:32 (24-mer-mouse (Form 2)). The sequences of such peptides are shown below in Table 1.

**Table 1.**

| SEQ ID NO | SEQUENCE | Description |
|---|---|---|
| 1 | MIETYSQPSP RSVATGLPAS MKIFMYLLTV FLITQMIGSV LFAVYLHRRL DKVEEEVNLH EDFVFIKKLK RCNKGEGSLS LLNCEEMRRQ FEDLVKDITL NKEEKKENSF EMQRGDEDPQ IAAHVVSEAN SNAASVLQWA KKGYYTMKSN LVMLENGKQL TVKREGLYYV YTQVTFCSNR EPSSQRPFIV GLWLKPSSGS ERILLKAANT HSSSQLCEQQ SVHLGGVFEL QAGASVFVNV TEASQVIHRV GFSSFGLLKL | SwissPro 27548.2 Mouse CD40 Ligand (CD154 Protein) |
| 2 | | SwissPro 29965 Human CD40 Ligand (CD154 Protein) |
| 3 | KGYY | Core-sequence |
| 4 | KKGYYT | 6-mer |
| 5 | AKKGYYTM | 8-mer-mouse |
| 6 | AEKGYYTM | 8-mer human |
| 7 | VLQWAKKGYYTMKSN | 15-mer-mouse |
| 8 | VLQWAEKGYYTMSNN | 15-mer human |
| 9 | NAASVLQW AKKGYYTMKSNL VMLE | 24-mer |
| 10 | ISQAVHAAHAEINEAGR | 15-mer from ovalbumin; control peptide |
| 11 | G-L-Q-W-A-K-K-G-Y-Y-T-M-K-S-N | Gly-1 |
| 12 | V-G-Q-W-A-K-K-G-Y-Y-T-M-K-S-N | Gly-2 |
| 13 | V-L-G-W-A-K-K-G-Y-Y-T-M-K-S-N | Gly-3 |
| 14 | V-L-Q-G-A-K-K-G-Y-Y-T-M-K-S-N | Gly-4 |
| 15 | V-L-Q-W-G-K-K-G-Y-Y-T-M-K-S-N | Gly-5 |
| 16 | V-L-Q-W-A-G-K-G-Y-Y-T-M-K-S-N | Gly-6 |
| 17 | V-L-Q-W-A-K-G-G-Y-Y-T-M-K-S-N | Gly-7 |
| 18 | V-L-Q-W-A-K-K-G-G-Y-T-M-K-S-N | Gly-8 |
| 19 | V-L-Q-W-A-K-K-G-Y-G-T-M-K-S-N | Gly-9 |
| 20 | V-L-Q-W-A-K-K-G-Y-Y-G-M-K-S-N | Gly-10 |
| 21 | V-L-Q-W-A-K-K-G-Y-Y-T-G-K-S-N | Gly-11 |
| 22 | ISQAVHAAHAEINEAGR | 15-mer from ovalbumin; control peptide |
| 23 | YVQGKANLKSKLMYT | Scrambled peptide |
| 24 | WAKKGYYTMK | 10-mer mouse |
| 25 | VLQWAKKGYYTMK | 13-mer mouse |
| 26 | AASVLQW AKKGYYTMKSNLVMLEN | 24-mer mouse |
| 27 | KGYYTM | 6-mer (Form 2) |
| 28 | AEKGYY | 6-mer (Form 3) |
| 29 | AKKGYY | 6-mer (Form 4) |
| 30 | AKGYYT | 6-mer (Form 5) |
| 31 | YKNVKQMAYWLTGKS | Scrambled peptide |
| 32 | AASVLQW AKKGYYTMKSNL VMLEN | 24-mer-mouse (Form 2) |

Interaction of a CD40 protein and a CD154 protein has been shown to occur at particular regions within each protein. The inventors have now shown that, surprisingly, a peptide comprising only a short portion of the CD154 region that interacts with CD40 is capable of binding to a CD40 protein, thereby modulating type 2 diabetes. Thus one embodiment hereof is the peptide for use comprising amino acid sequence SEQ ID NO. 3, that comprises at least a portion of the amino acid sequence of a CD154 protein such that the peptide interacts with CD40 protein in such a manner as to modulate type 2 diabetes. In one embodiment, interaction of the peptide for use with CD40 protein results in negative, reduction, or inhibition of type 2 diabetes. In one embodiment, the peptide for use comprising amino acid sequence SEQ ID NO. 3, comprises at least a portion of SEQ ID NO:1 or SEQ ID NO:2.

In one embodiment, the peptide for use comprising the amino acid sequence SEQ ID NO. 3 is as short as possible yet comprises enough of the CD154 protein to allow interaction with a CD 40 protein in such a manner as to modulate type 2 diabetes. In one embodiment, the peptide for use hereof comprising the amino acid sequence SEQ ID NO. 3 comprises 6, 13 or 15 contiguous amino acids from SEQ ID NO:1 or SEQ ID NO:2, and interacts with CD40 in such a manner as to modulate type 2 diabetes. The peptide for use comprises a core sequence of lysine-glycine-tyrosine-tyrosine (KGYY; SEQ ID NO:3), which corresponds to amino acids 142-145 of SEQ ID NO:1 and amino acids 143-146 of SEQ ID NO:2. Moreover, a preferred peptide for use comprises a core sequence of lysine-glycine-tyrosine-tyrosine-threonine-methionine (KGYYTM; SEQ ID NO:27), which corresponds to amino acids 142-147 of SEQ ID NO:1 and amino acids 143-148 of SEQ ID NO:2. Useful peptides can comprise additional regions of sequence from SEQ ID NO:1 or SEQ ID NO:2 that are adjacent to the core sequence, so long as the peptide is capable of modulating type 2 diabetes. In one embodiment hereof, the peptide for use comprises at least one sequence selected from SEQ ID NO:3, SEQ ID NO:4, SEQ ID NO:7, SEQ ID NO:8, SEQID NO:9, SEQ ID NO:27, SEQ ID NO:28, SEQ ID NO:29, SEQ ID NO:30 and SEQ ID NO:32 so long as the peptide interacts with CD40 protein in such a manner as to modulate type 2 diabetes. In one embodiment of the present subject matter, the peptide for use hereof is a sequence selected from SEQ ID NO:3, SEQ ID NO:4, SEQ ID NO:7, SEQ ID NO:8, SEQID NO:9, SEQ ID NO:27, SEQ ID NO:28, SEQ ID NO:29, SEQ ID NO:30 and SEQ ID NO:32.

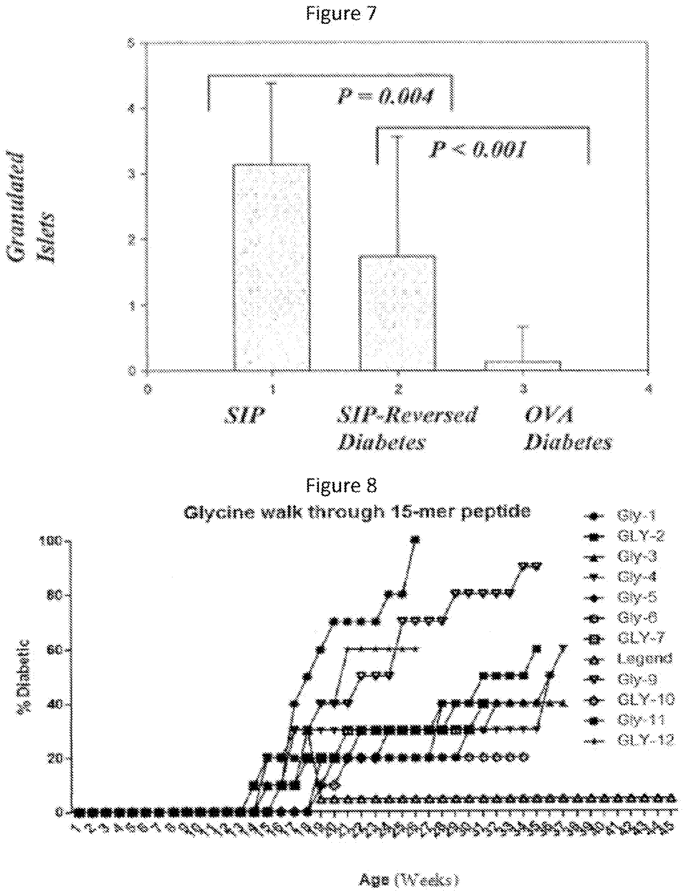

While peptides of the present subject matter can be selected entirely of or from sequences that are responsible for the interaction of the peptide with a CD40 protein, they may additionally contain amino acid sequences that do not interact with a CD40 protein, but which have other useful functions. Any useful, additional amino acid sequence can be added to the CD40-interacting sequence, so long as the additional sequences do not have an unwanted effect on the ability of the CD40 interacting sequence to interact with a CD40 protein. For example, in addition to the amino acid sequence responsible for interacting with a CD40 protein, a peptide hereof can contain amino acid sequences that are useful for visualizing or purifying the peptide. Such sequences act as labels (e.g., enzymes) or tags (antibody binding sites). Additionally, the developments presented herein demonstrate that substitutions of amino acids at any position other than the position-7 (K) of the 15-mer and the position-9 (Y) of the 15-mer may be made and the integrity and function of the peptide may be maintained (see Fig. 8). Accordingly, these developments may contemplate that numerous substitutions may be possible while still maintaining the beneficial aspects of the peptide.

Any useful, additional amino acid sequence can be added to the CD40-interacting sequence, so long as the additional sequences do not have an unwanted effect on the ability of the CD40 interacting sequence to interact with a CD40 protein. For example, in addition to the amino acid sequence responsible for interacting with a CD40 protein, a peptide hereof can contain amino acid sequences that are useful for visualizing or purifying the peptide. Examples of such labels and tags include, but are not limited to, B-galactosidase, luciferase, glutathione-s-transferase, thioredoxin, HIS-tags, biotin tags, and fluorescent tags. Moreover, acetyl groups and amides may be appended on the N-terminus or C-terminus, and the developments hereof contemplate these and other variations that may enhance stability or other traits desired of such a peptide. Other useful sequences for labeling and tagging proteins are known to those of skill in the art.

Likewise, peptides hereof can be modified, so long as such modification does not significantly affect the ability of the peptide to modulate type 2 diabetes. Such modifications can be made, for example, to increase the stability, solubility or absorbability of the protein. Examples of such modifications include, but are not limited to pegylation, glycosylation and chemical modification of the peptide.

Peptides hereof may possibly be derived from nature (e.g., obtained from plants, animals or microorganisms) or they can be produced in a laboratory (e.g., recombinantly or synthetically). Preferred peptides for use are those that are synthesized. Also encompassed are peptides for use that are combinations of natural and synthetic molecules. General methods for producing and isolating recombinant or synthetic peptides are known to those skilled in the art. It should be noted that, as used herein, an isolated, or biologically pure, molecule, is one that has been removed from its natural milieu. As such the terms isolated, biologically pure, and the like, do not necessarily reflect the extent to which the protein has been purified.

The peptides hereof do not arise naturally in a corporeal body, but rather must be constructed and synthesized to obtain the small interfering peptides. Certain aspects of the design and synthesis may affect the peptides stability and ability to perform its intended use. The peptides hereof may vary in length from four amino acids in length as in SEQ ID NO:3, five amino acids in length, six amino acids in length as in SEQ ID NOs: 4, 27, 28, 29, and 30, seven amino acids in

length, eight amino acids in length as in SEQ ID NOs: 5 and 6, nine amino acids in length, ten amino acids in length as in SEQ ID NOs: 24, eleven amino acids in length, twelve amino acids in length, thirteen amino acids in length as in SEQ ID NO: 25, fourteen amino acids in length, fifteen amino acids in length as in SEQ ID NOs: 7, 8, 11, 12, 13, 14, 15, 16, 17, 18, and 20, sixteen amino acids in length, seventeen amino acids in length, eighteen amino acids in length, nineteen amino acids in length, twenty amino acids in length, twenty-one amino acids in length, twenty-two amino acids in length, twenty-three amino acids in length, twenty-four amino acids in length as in SEQ ID NO: 26 and 32, and twenty-five amino acids in length.

As has been described herein, interaction of the CD40 protein and the CD154 protein are necessary for involvement of Th40 cells in type 2 diabetes. Consequently, inhibition of the interaction between a CD40 and CD154 protein using peptides hereof is a useful method of affecting type 2 diabetes. In one aspect hereof, the peptide for use reduces the interaction between the CD40 protein and the CD154 protein by at least 5%, at least 10%, at least 20%, at least 30%, at least 40%, at least 50%, at least 60%, at least 70%, at least 80%, at least 90%, or at least 95%. In one embodiment, the peptide for use reduces the interaction between the CD40 protein and the CD154 protein by a factor of at least 10, at least 100, at least 1,000, at least 10,000. Methods of measuring the strength of the interaction between the CD40 protein and the CD154 protein have been discussed previously, and are also know to those of skill in the art.

One embodiment is the peptide for use hereof in a method to reduce type 2 diabetes in a patient, the method comprising administering the peptide hereof to the patient. In one embodiment, the peptide for use comprises an amino acid sequence selected from SEQ ID NO:3, SEQ ID NO:4, SEQ ID NO:5, SEQ ID NO: 6, SEQ ID NO:7, SEQ ID NO:8, SEQ ID NO:9, SEQ ID NO:25, SEQ ID NO:26, SEQ ID NO:27, SEQ ID NO: 28, SEQ ID NO: 29, SEQ ID NO:30 and SEQ ID NO:32. In one embodiment, the peptide for use is an amino acid sequence selected from SEQ ID NO:4, SEQ ID NO:8, SEQ ID NO:9, SEQ ID NO:27, SEQ ID NO: 28, and SEQ ID NO: 29. In a preferred embodiment, interaction of the peptide for use with the CD40 protein decreases the number of Th40 cells by at least 5%, at least 10%, at least 20%, at least 30%, at least 40%, at least 50%, at least 60%, at least 70%, at least 80%, at least 90%, or at least 95%. In another embodiment, interaction of the peptide for use with the CD40 protein decreases the number of Th40 cells by a factor of at least 10, at least 100, at least 1,000, at least 10,000. In a preferred embodiment, the level of Th40 cells is reduced so that Th40 cells comprise no more than about 20%, about 25%, about 30%, about 35%, or about 40% of the total T-cell population.

Peptides for use hereof are suitable for use in cell culture as well as for treating a patient. As used herein the term patient refers to any animal in need of such treatment. The animal can be a human or a non-human animal. A preferred animal to treat is a mammal. A peptide can be administered or applied per se, or as pharmaceutical compositions. A peptide hereof, or a pharmaceutical composition thereof, can be administered to a patient by a variety of routes, including, but limited to, by injection (e.g., intravenous, intramuscular, subcutaneous, intrathecal, intraperitoneal), by inhalation, by oral (e.g., in a pill, tablet, capsule, powder, syrup, solution, suspension, thin film, dispersion or emulsion.), transdermal, transmucosal, pulmonary, buccal, intranasal, sublingual, intracerebral, intravaginal rectal or topical administration or by any other convenient method known to those of skill in the art.

The amount of a peptide hereof and/or a pharmaceutical composition thereof that will be effective can be determined by standard clinical techniques known in the art. Such an amount is dependent on, among other factors, the patient being treated, including, but not limited to the weight, age, and condition of the patient, the intended effect of the compound, the manner of administration and the judgment of the prescribing physician. Also, in this context, it should be noted that in treating a patient exhibiting a disorder of interest, a therapeutically effective amount of an agent or agents such as these is administered. A therapeutically effective dose refers to that amount of the compound that results in amelioration of one or more symptoms or a prolongation of survival in a patient.

A peptide hereof, or a pharmaceutical composition thereof, can be administered alone or in combination with one or more other pharmaceutical agents, including other compounds of the present disclosure. The specific pharmaceutical composition depends on the desired mode of administration, as is well known to the skilled artisan.

Because the inventors have discovered that Th40 cells are intimately involved in the development of autoimmune diseases and type 2 diabetes, the peptides and methods disclosed herein can be used to affect conditioning resulting from such diseases. Thus, one embodiment hereof is the peptide for use in a method to treat type 2 diabetes in a patient in need of such treatment, the method comprising administering to a patient the peptide that interacts with the CD40 protein, thereby reducing type 2 diabetes. In one embodiment the peptide for use interacts with the CD40 protein in such a manner as to affect the interaction of CD40 and CD154, thereby reducing type 2 diabetes. In a preferred embodiment, interaction of the peptide for use with the CD40 protein reduces the number of Th40 cells in a patient to a level equal to that observed in subjects that do not have type 2 diabetes. In another embodiment, interaction of the peptide for use with the CD40 protein reduces the inflammatory cytokine levels in a patient. In another embodiment, interaction of the peptide for use with the CD40 protein reduces the inflammatory cytokine levels in a patient to a level equal or similar to those observed in subjects that do not have type 2 diabetes.

In type 2 diabetes, glucose tolerance is reduced, insulin sensitivity is decreased, and plasma insulin levels are increased. Consequently, control of inflammatory cells and cell signaling via CD40-CD154 interaction may be able to be used to control, modulate, reduce and/or reverse type 2 diabetes symptoms that are characterized as by glucose intolerance, insulin resistance, and increased plasma insulin levels. Several murine models of T2D and/or atherosclerosis have been developed. For initial studies, ApoE-/- mice were selected due to their ability to develop type 2 diabetes from a high fat diet. Glucose tolerance and insulin testing were performed on all of the mice. The mice were administered the 6-mer peptide (SEQ ID NO:29) at a rate of 1mg/kg weekly via I.V. injection and monitored. Peptide treated ApoE deficient mice demonstrated significantly increased glucose tolerance as well as significantly improved insulin sensitivity, improve insulin resistance, and lowered plasma insulin levels compared to controls. Thus, one embodiment of the present developments is addressed to the peptides for use in a method to prevent type 2 diabetes in an individual at risk for developing type 2 diabetes, the method comprising administering to the individual the peptide as defined herein to selectively bind to a CD40 expressing cell. In such an embodiment, the peptide for use may be selected from SEQ ID NOS: 3-9 and 24-30.

The developments hereof have also shown that, surprisingly, peptides hereof can be used to reverse the disease process in individuals already showing signs of type 2 diabetes. Thus, one embodiment of the present subject matter is addressed to the peptide for use in a method to reverse type 2 diabetes comprising administering to a patient diagnosed as having type 2 diabetes, the peptide hereof. In one embodiment, the peptide for use comprises an amino acid sequence selected from SEQ ID NO:3, SEQ ID NO:4, SEQ ID NO:7, SEQ ID NO:8, SEQ ID NO:9, SEQ ID NO:27, SEQ ID NO:28, SEQ ID NO:29, and SEQ ID NO:30, so long as the peptide can down-regulate or reduce inflammation. In one embodiment, the peptide for use is an amino acid sequence selected from SEQ ID NO:3, SEQ ID NO:4, SEQ ID NO:7, SEQ ID NO:8, SEQ ID NO:9, SEQ ID NO:27, SEQ ID NO:28, SEQ ID NO:29, and SEQ ID NO:30. As used herein the phrase to reverse type 2 diabetes means to increase glucose tolerance, decrease insulin resistance, and decrease plasma insulin levels to levels closer to or more comparable to those observed in individuals who do not have type 2 diabetes.

In yet another development hereof has shown that, surprisingly, peptides hereof can be used to reverse the disease process in individuals already showing signs of type 2 diabetes. Thus, one embodiment of the present subject matter is addressed to the peptide for use in a method to reverse type 2 diabetes comprising administering to a patient diagnosed as having type 2 diabetes, the peptide hereof. In one embodiment, the peptide for use comprises an amino acid sequence selected from SEQ ID NOS: 3-9 and 24-30, so long as the peptide can control, reduce and/or reverse inflammation. In one embodiment, the peptide for use is an amino acid sequence selected from SEQ ID NOS: 3-9 and 24-30, so long as the peptide can control, reduce and/or reverse type 2 diabetes. In an embodiment, interaction of the peptide for use with the CD40 protein reduces the number of Th40 cells in a patient to a level equal to that observed in subjects that do not have type 2 diabetes. In another embodiment, interaction of the peptide for use with the CD40 protein reduces the inflammatory cytokine levels in the patient. In another embodiment, interaction of the peptide for use with the CD40 protein reduces the inflammatory cytokine levels in the patient to a level equal or similar to those observed in subjects that do not have type 2 diabetes.

As has been described, peptides of the present invention selectively bind to a CD40 expressing cell. Consequently, peptides of the present subject matter can be used to identify Th40 cells. Such detection can be performed using assay techniques known to those skilled in the art. In general, an assay for detecting Th40 cells using a peptide hereof comprises (a) obtaining a sample of cells; (b) contacting a peptide hereof with said cells under condition suitable to allow binding of the peptide to Th40 cells, if present; (c) washing said cells using conditions that disrupt non-specific interactions, and that remove unbound peptide; and (d) detecting peptide bound to cells. Detection of bound peptide can be achieved directly or indirectly. For example, direct detection can be achieved using a peptide labeled using a detectable marker, as disclosed herein. Following the wash step listed above, the cells are then simply screened for the presence of detectable marker. The presence of detectable marker in the cell sample indicates the presence of Th40 cells, and thus Th40-dependent type 2 diabetes. Alternatively, indirect detection involves the use of a second molecule, such as an antibody, that binds to the peptide. In an indirect detection assay, following the wash step listed above, a detection molecule that binds the peptide is added to the cell sample. The detection molecule is labeled with a detectable marker. After washing away unbound detection molecule, the cells are screened for the presence of detectable marker. The presence of detectable marker in the cell sample indicates the presence of Th40 cells. It should be understood that the assays described herein are meant as examples of useful assays, and other assay techniques can be employed. Suitable assay techniques are known to those skilled in the art, and are also disclosed in, for example, Molecular Cloning: A Laboratory Manual, Sambrook, J., Fritsch, E.F., and Maniatis, T, Cold Spring Harbor Laboratory Press; 2nd Edition (December 1989).

The assay technology described above can also be used to identify other molecules that affect the interaction of a CD40 protein with a CD514 protein. Examples of such molecules include, but are not limited to, proteins, peptides and small molecules. For example, assays can be designed that test the ability of molecules to compete with a peptide of the present developments for binding to a Th40 cell. For instance, a peptide labeled with a detectable marker, can be mixed with a test molecule and a population of cells known to contain Th40 cells, under conditions that allow binding of the peptide to the Th40 cells. Following an appropriate incubation period, the cells are washed to remove unbound peptide, and the cells screened for the presence of detectable marker. Alternatively, the labeled peptide could be bound to Th40 cells first, and after a wash step to remove unbound peptide, the test molecule could be added to the cells containing bound peptide. Following an incubating period and a wash step to remove unbound molecule, or released peptide, the cells are screened for the presence of detectable marker. In either case, absence of the detectable marker in the cell sample indicates the test molecule is able to compete with the peptide for binding to the Th40 cells, while presence of the detectable marker would indicate the test molecule does not inhibit binding of the peptide to Th40 cells. Inhibition of binding need not be 100%, as such assay would also be useful for identifying molecules that partially inhibit binding of the peptide to Th40 cells. It is understood by those skilled in the art that such assays would involve the use of positive controls (e.g., unlabeled peptide) and negative controls (e.g., a protein/molecule that is known not to bind to Th40 cells).

The assay technology above can also be used to identify other molecules that affect the interaction of a CD40 protein with a CD154 proteins. Examples of such molecules include, but are not limited to, proteins, peptides and small molecules. For example, assays can be designed that test the ability of molecules to compete with a peptide of the present developments for binding to a CD40 protein of cells other than T cells, such as neutrophils, eosinophils, basophils, mast cells, macrophages, platelets, endothelial cells, and lymphocytes, including natural killer cells and B cells. For instance, a peptide labeled with a detectable marker, can be mixed with a test molecule and a population of cells known to contain CD40 containing cells, under conditions that allow binding of the peptide to the CD40 bearing cells. Following an appropriate incubation period, the cells are washed to remove unbound peptide, and the cells screened for the presence of detectable marker. Alternatively, the labeled peptide could be bound to CD40 bearing cells first, and after a wash step to remove unbound peptide, the test molecule could be added to the cells containing bound peptide. Following an incubating period and a wash step to remove unbound molecule, or released peptide, the cells are screened for the presence of detectable marker. In either case, absence of the detectable marker in the cell sample indicates the test molecule is able to compete with the peptide for binding to the CD40 bearing cells, while presence of the detectable marker would indicate the test molecule does not inhibit binding of the peptide to CD40 bearing cells. Inhibition of binding need not be 100%, as such assay would also be useful for identifying molecules that partially inhibit binding of the peptide to CD40 bearing cells. It is understood by those skilled in the art that such assays would involve the use of positive controls (e.g., unlabeled peptide) and negative controls (e.g., a protein/molecule that is known not to bind to CD40 bearing cells).

Because increased levels of Th40 cells are associated with the development of autoimmune disease, the present developments can be used to identify patients at risk for developing autoimmune disease and autoimmune related type 2 diabetes.

The following examples are provided for the purposes of illustration and are not intended to limit the scope of the present invention.

### Examples

### Example 1

This Example demonstrates the effect of various peptide fragments of CD154 on CD4/CD8 ratios and the development of diabetes in NOD mice.

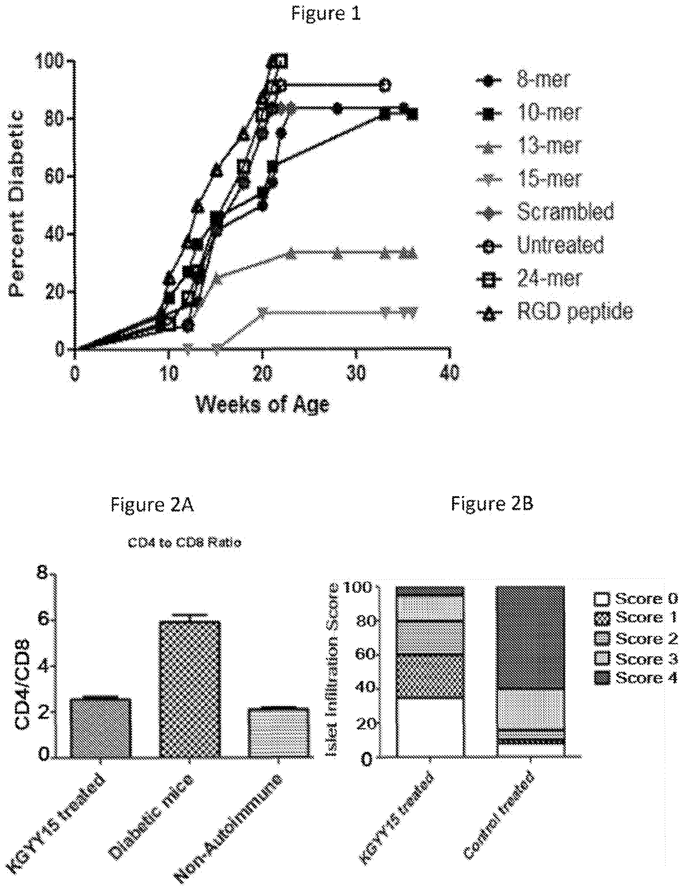

Peptides were designed based on the amino acid sequence of mouse CD154 protein (SEQ ID NO:1) in the SwissPro database. The peptides (8-mer (SEQ ID NO: 5; SEQ ID NO: 6), 10-mer (SEQ ID NO:24), 13-mer (SEQ ID NO:25), 15-mer (SEQ ID NO: 7), 24-mer (SEQ ID NO:26), scrambled (SEQ ID NO: 23), and RGD (arginylglycylaspartic acid) were then ordered from New England Peptide. The RGD peptide is a 15-amino acid sequence from the CD154 sequence that does not include the CD40 binding motif. The lyophilized peptides were suspended in sterile saline at 1 mg/ml. 25 ug in 100ul (1mg/kg) of a particular peptide was then injected into the tail vein of 6-week old NOD mice. Control mice received 100 ul of sterile saline. This is well before the onset of diabetes (and atherosclerosis), but after damage to pancreatic islets has begun. Weekly after the initial injection, another 25 ug of peptide (or 100 ul of saline in the case of the Control mice) was injected into the tail vein. At 10 weeks of age, mice were monitored for diabetes, as indicated by a blood glucose level greater than 250 mg/dL for three consecutive days. The results of this study are shown in Figure 1. During this time, blood was also taken from the tail vein, or by sub-mandibular venal puncture, and the level of CD4+ and CD8+ cells determined by flow cytometry using antibodies for CD4 protein and CD8 protein. The results of this analysis are shown in Figure 2A.

Pancreata were excised and examined by histology for cellular infiltrates and assigned scores based on observable, measurable, and quantifiable data: 0 = no infiltrate; 1 = one pole infiltrate; 2 = peri-insulitis, bi-polar-infiltrates; 3 = 75% infiltrate and 4 = full infiltration. The results of this analysis are shown in Figure 2B.

The results demonstrate that treatment with a peptide unrelated to the CD154 protein did not reduce the development of diabetes in NOD mice. In contrast, treatment of mice with a 15-mer peptide derived from the CD154 protein prevented the onset of diabetes. Further, the 13-mer peptides derived from the CD154 protein had significant effects on the development of diabetes. In addition, the data demonstrates that the 15-mer peptide did not result in compromise of the immune system, as determined by the CD4/CD8 ratio.

### Example 2

This Example demonstrates the effect of the 15-mer peptide on hyperglycemia in newly diabetic NOD mice.

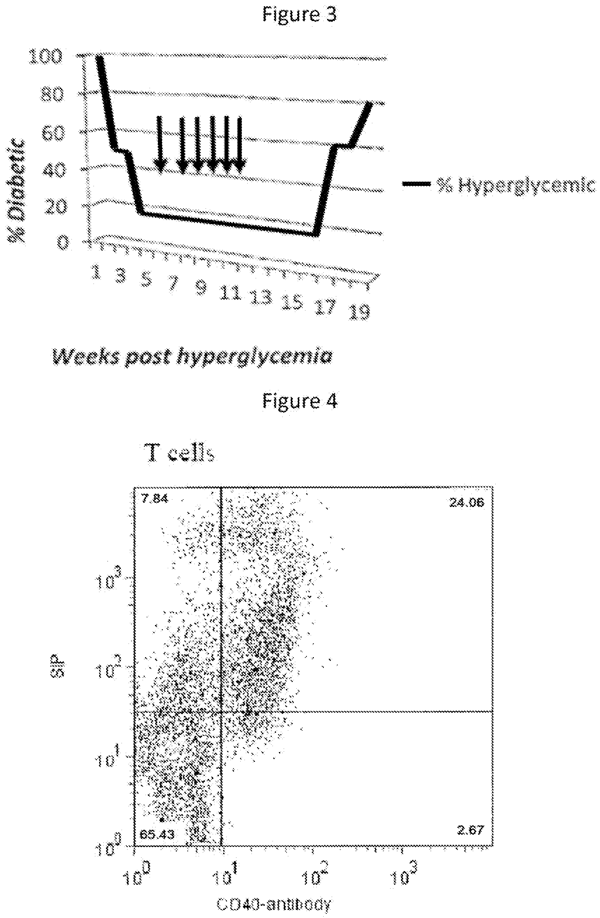

Six mice from Example 1 that were not treated were allowed to subsequently develop diabetes. These mice were injected intravenously with 100 ug of the 15-mer peptide. These mice were then given weekly injections of the 15-mer peptide into their tail veins, and their blood glucose levels monitored twice-weekly. The 15-mer peptide was administered for a total of ten weeks, after which the treatment was stopped. The results of this study are shown in Figure 3.

This study demonstrates that injection of the 15-mer peptide into already diabetic mice can reverse hyperglycemia. It also demonstrates that cessation of the treatment results in return of hyperglycemia within 5 weeks.

### Example 3

This study demonstrates the ability of the 15-mer peptide to bind to Th40 cell and B cells.

Total lymphocytes were isolated from 9 week old NOD mice. The lymphocytes were incubated with anti-CD4, anti-CD8, anti-CD40 and an FITC-labeled 15-mer peptide, and then analyzed by flow cytometry. Cells were gated for CD4 (both CD4hi and CD4lo populations were included) and CD40 versus the 15-mer peptide. The results of this analysis are shown in Figure 4.

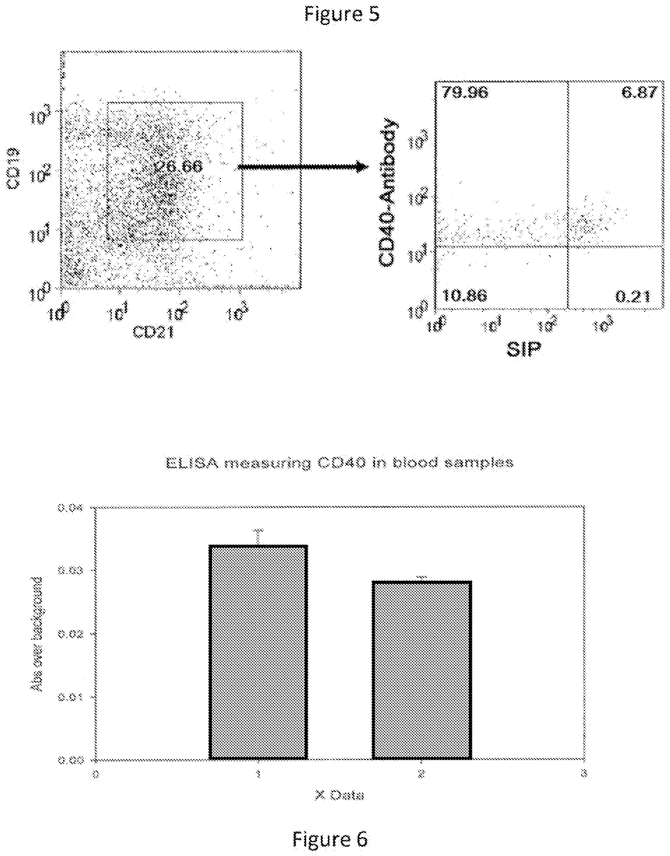

B cells were isolated from the spleens of NOD mice. Sorted MHC-II+ cells were purified from total lymphocytes. Cells were stained with FITC-labeled 15 mer peptide, anti-CD40, and B cell markers CD19 and CD21. MHC-II+ cells were gated for CD19+ and CD21+ and then 15-mer peptide versus CD40 antibody was measured. The results of this study are shown in Figure 5.

This study shows that a substantial majority, 90% of CD40+ T-cells, also bind the 15-mer peptide, thereby demonstrating that the 15-mer peptide is highly specific for CD40+ cells. It also shows that while 90% of B cells were CD40 positive, only 8% of B cells bound the 15-mer peptide.

### Example 4

This example demonstrates the level of CD40 positive cells in the blood of type-I diabetic subjects and non-diabetic (control) subjects.

1 ml of whole blood was obtained from each individual and incubated with biotin-conjugated, 15-mer peptide. The cells were then exposed to horseradish peroxidase (HRP)-avidin, washed and the absorbance at 405 nm determined using a spectrophotometer. The results of this study are shown in Figure 6.

This study demonstrates that blood cells from patients having type-I diabetes had higher 15-mer peptide binding activity than cells from non-diabetic controls.

### Example 5

This example demonstrates the level of insulin granulation observed in the pancreas of NOD mice treated with either the 15-mer peptide or a peptide from ovalbumin.

At the onset of diabetes, six NOD mice were injected with 100 ug/ml of the 15-mer peptide, resulting in the reversal of hyperglycemia in 80% of the recipients. Six weeks after reversal of hyperglycemia, mice were sacrificed, and the pancreas removed for analysis. The pancreas was fixed, sectioned and then stained using an aldehyde/fuschsin stain that allows detection of insulin granules. Granulation of the tissue was scored as follows: 4=completely granulated; 3=75% of islet granulated; 2= 50% of islet granulated, and peri-insulitis; 1=25% of islet granulated; 0= no insulin granules detected. The results of this analysis are shown in Figure 7.

This analysis demonstrates that the 15-mer peptide preserved insulin granules in the majority of the mice, and was significantly improved in peptide-reversed diabetic mice compared to diabetic mice that received an irrelevant peptide.

### Example 6

This example demonstrates the effect of mutations in the 15-mer peptide on its ability to prevent the onset of diabetes. Fig. 8 provides results related to this Example 6.

Peptide were designed and produced as described in Example 1. Variant peptides were produced so that in each variant, a glycine was substituted for an amino acid corresponding to an amino acid in positions 1-7 or 9-12 of SEQ ID NO:7, as follows:
Gly-1 G-L-Q-W-A-K-K-G-Y-Y-T-M-K-S-N (SEQ ID NO:11)
Gly-2 V-G-Q-W-A-K-K-G-Y-Y-T-M-K-S-N (SEQ ID NO:12)
Gly-3 V-L-G-W-A-K-K-G-Y-Y-T-M-K-S-N (SEQ ID NO:13)
Gly-4 V-L-Q-G-A-K-K-G-Y-Y-T-M-K-S-N (SEQ ID NO:14)
Gly-5 V-L-Q-W-G-K-K-G-Y-Y-T-M-K-S-N (SEQ ID NO:15)
Gly-6 V-L-Q-W-A-G-K-G-Y-Y-T-M-K-S-N (SEQ ID NO:16)
Gly-7 V-L-Q-W-A-K-G-G-Y-Y-T-M-K-S-N (SEQ ID NO:17)
Gly-9 V-L-Q-W-A-K-K-G-G-Y-T-M-K-S-N (SEQ ID NO:18)
Gly-10 V-L-Q-W-A-K-K-G-Y-G-T-M-K-S-N (SEQ ID NO:19)
Gly-11 V-L-Q-W-A-K-K-G-Y-Y-G-M-K-S-N (SEQ ID NO:20)
Gly-12 V-L-Q-W-A-K-K-G-Y-Y-T-G-K-S-N (SEQ ID NO:21)

NOD mice were placed in groups of 10, and the mice in each group injected IV weekly with 25 ug of either wild-type (WT; Legend) peptide or a variant peptide (in PBS, ph 7.2) listed above. The development of diabetes was monitored by measuring blood glucose levels on a weekly basis. Mice were considered "diabetic" when blood glucose was 250 mg/dl or greater for 3 consecutive readings. Injections began at 6 weeks of age = pre-diabetes.

This example demonstrates that substitution of a glycine at any of positions 1-7, or 9-12, reduces the ability of the 15-mer peptide to inhibit the development of diabetes. It also shows that such mutations do not completely abolish the ability of the mutated 15-mer peptide to inhibit the development of diabetes. Substitutions at the Tyr position 9, Thr position 11, and Met position 12 proved differential for hyperglycemic prevention activity, as shown in Fig. 8. Therefore it is also postulated that a second 6-mer derivative - SEQ ID NO:27 - 6-mer (Form 2), SEQ ID NO:28 - 6-mer (Form 3), SEQ ID NO:29 - 6-mer (Form 4), and SEQ ID NO:30 - 6-mer (Form 5) may also provide increased therapeutic efficacy.

### Example 7

This example demonstrates that the same elevation of Th40 cell levels in the ApoE deficient mouse model of atherosclerosis is also notably elevated in human Type 1 Diabetes (T1D).

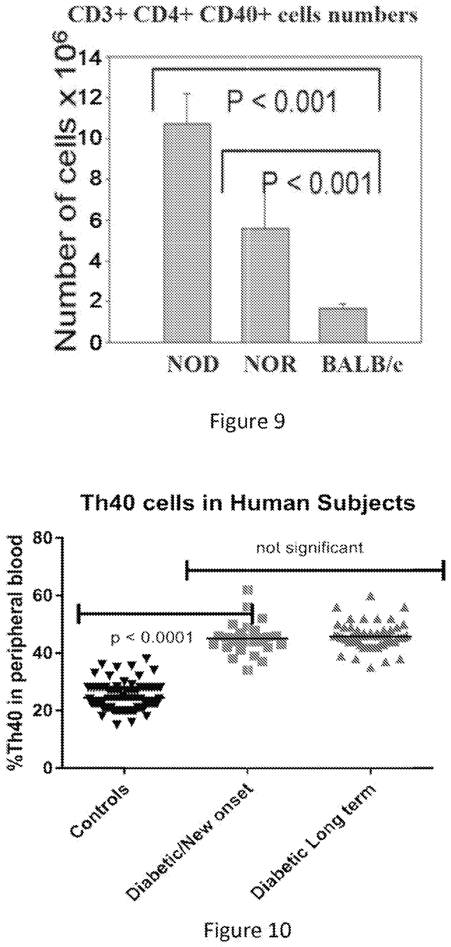

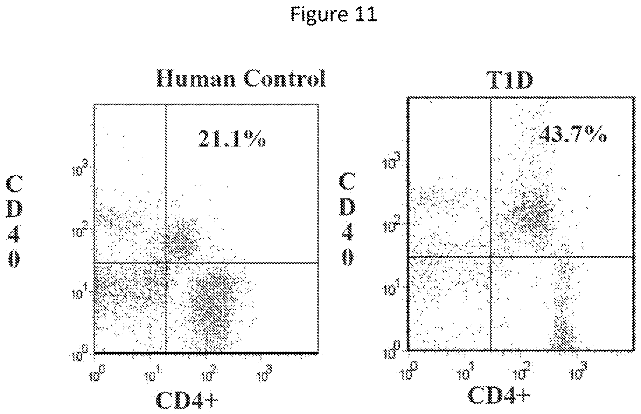

The peripheral blood was measured was measured for total count of CD3+CD4+CD40+ cell numbers in NOD, NOR (non-obese diabetic resistant), and BALB/c (control) mice as in Figure 9. This was compared to the percentage of Th40 cells in peripheral blood in human subjects for control, diabetic/new onset, and long term diabetic populations as in Figure 10. Further, lymphocytes were isolated from 9-week old NOD mice. The lymphocytes were incubated with anti-CD, anti-CD8, and an FITC-labeled 15-mer peptide, and then analyzed by flow cytometry. Cells were gated for CD4 (both CD4hi and CD4lo populations were included) and CD4 versus the 15-mer peptide. These results are displayed in Figure 11.

ApoE deficient mice on a normal chow diet were selected to receive a dose of 1mg/kg of the 15-mer peptide (SEQ ID NO: 7) by IV tail injection, three times a week over a period of 26 weeks, beginning at 9 weeks of age and also utilized a control. At 25 weeks, the animals were euthanized, weighed, and then had blood, spleen, and pancreas removed for analysis. The subjects were then perfused through cardiac puncture with 4% paraformaldehyde. Aortic arches were dissected, dehydrated in sucrose gradient and then flash frozen. Approximately thirty-five 8um longitudinal sections were obtained per mouse for various staining procedures. Flow cytometry was performed utilizing a MACSQuant^{®} Analyzer 10 (Miltenyi Biotec Inc.). Additional analysis was performed using FlowJo ^{®} (FlowJo, LLC wholly owned by BectonDickinson, Inc.) single-cell flow cytometry software.

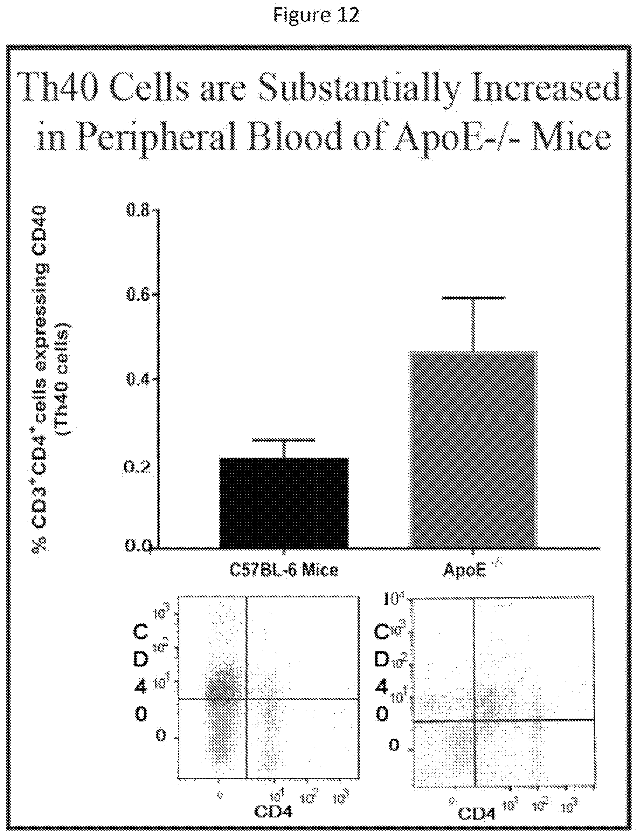

C57BL/6 and ApoE-/- mice demonstrated increased levels of Th40 cells relative to all CD3+CD4+ cells prior to hyperglycemia as demonstrated in Figure 12.

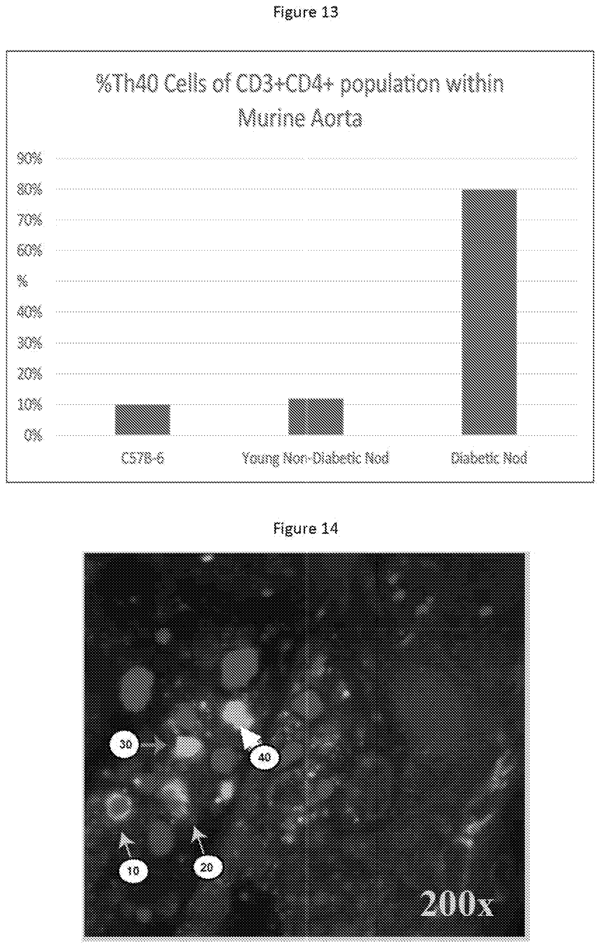

Further, NOD mice tested in this study demonstrated significant Th40 infiltration in the aorta compared with control and young non-diabetic NOD mice populations, as shown in Figure 13.

An exemplar aortic plaque of one of the longitudinal sections was observed at 200x magnification using oil-red-0, trichrome stain and immune-fluorescence, and is shown in Figure 14. This microscopy and stain of the aorta showed that not only are the Th40 cells increased in the aorta similarly to the peripheral blood as demonstrated in Figs. 10-14, but also the Th40 cells are found within the shoulder region of plaque in the ApoE-/-model (the growth region of plaque/atherosclerosis). In Figure 14, **10** and **20** identify cells that represent CD3+, CD4+, and CD40+ (Th40 cells) that have significant intracellular CD40. **30** demonstrates Th40 cell with extracellular expression and no demonstrative CD40 intracellularly. **40** identifies CD3+, CD4+, CD40neg cell.

### Example 8

This example demonstrates that CD3+CD4+CD40+ cells appear to produce interferon gamma (INFγ) in abundance. Additionally, interferon gamma controls Th40 proliferation.

ApoE deficient mice on a normal chow diet were selected to receive a dose of 1mg/kg of the 15-mer peptide (SEQ ID NO: 7) by IV tail injection, three times a week over a period of 26 weeks, beginning at 9 weeks of age and also utilized a control. At 25 weeks, the animals were euthanized, weighed, and then had blood, spleen, and pancreas removed for analysis. The subjects were then perfused through cardiac puncture with 4% paraformaldehyde. Aortic arches were dissected, dehydrated in sucrose gradient and then flash frozen. Approximately thirty-five 8µm longitudinal sections were obtained per mouse for various staining procedures. Flow cytometry was performed utilizing a MACSQuant^{®} Analyzer 10 (Miltenyi Biotec Inc.). Additional analysis was performed using FlowJo ^{®} (FlowJo, LLC wholly owned by BectonDickinson, Inc.) single-cell flow cytometry software.

As demonstrated in Figure 14 through confocal microscopy, CD40 can be internal or external to the CD3+CD4+ cell. Flow cytometry was further performed and demonstrated that while most CD3+ cells appear to have ability to produce CD40, the CD3+CD4+CD40+ cells appear to produce interferon gamma (IFNγ) in abundance. This flow cytometry study incorporated both the external and internal staining of CD3, CD4, CD40, and IFNγ.

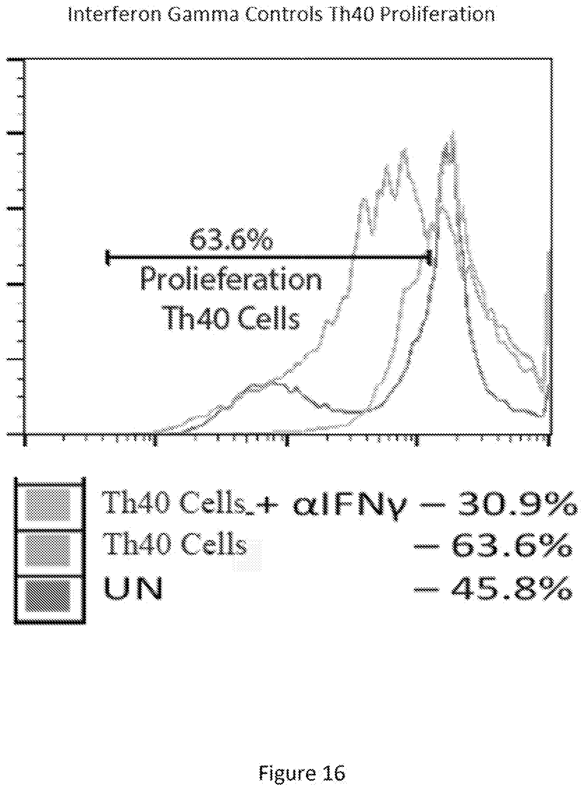

Figure 16 demonstrates that interferon gamma controls Th40 proliferation. Isolated Th40 cells were cross-linked by antibody to CD40. The graph in Figure 16 denotes proliferation of CD40 stimulated (CD40XL: activated) Th40 cells in the absence/presence of antibody to INFγ (αIFNγ) and non-stimulated controls (UN). Additionally, by blocking IFNγ, activated Th40 cells do not proliferate.

### Example 9

This example demonstrates that KGYY₁₅ (15-mer - SEQ ID NO:7) and KGYY₆ (6-mer - SEQ ID NO:29) abrogates atherosclerosis.

ApoE deficient mice on a normal chow diet were selected to receive a dose of 1mg/kg of the 15-mer peptide (SEQ ID NO: 7) by IV tail injection, three times a week over a period of 26 weeks, beginning at 9 weeks of age and also utilized a control. At 25 weeks, the animals were euthanized, weighed, and then had blood, spleen, and pancreas removed for analysis. The subjects were then perfused through cardiac puncture with 4% paraformaldehyde. Aortic arches were dissected, dehydrated in sucrose gradient and then flash frozen.

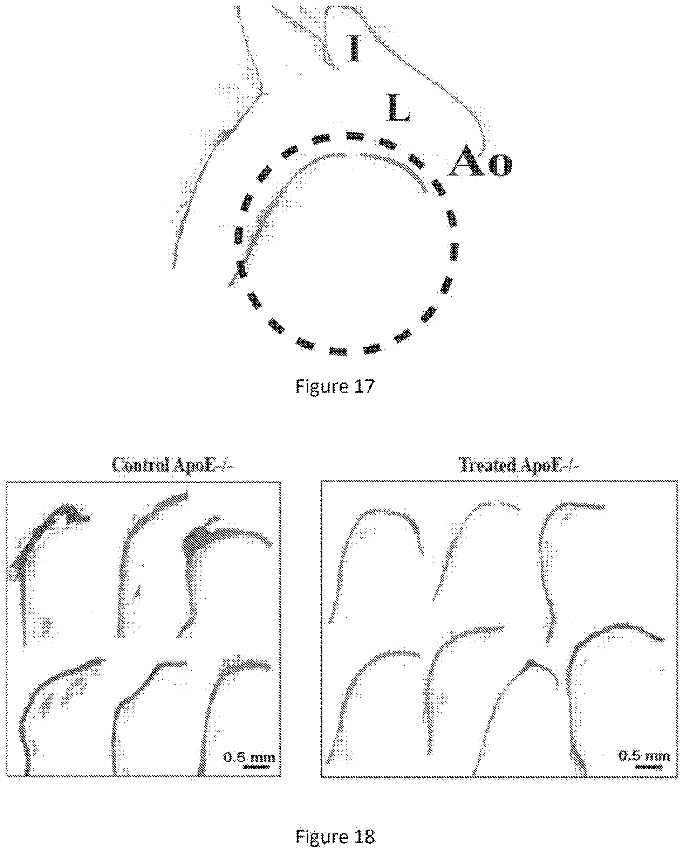

Figure 17 provides an example of the lesser curvature of the aortic arch, defined proximally from the aortic outflow (AO). Of the segments seen in the trichrome stain, an intimal distance of 2.4 mm was measured distally. The aortic-arch wall area subtended by this 2.5 mm stretch of the intima was calculated for each section of all mice, with maximal area of the inner-aortic-arch wall of each mouse used to compute averages per group. The luminal surface (L), aortic arch (AO), and innominate artery (I) are labelled in this Figure 17.

Figure 18 demonstrates the lesser curvature of the aortic arch of the control ApoE mice compared to the lesser curvature of the aortic arch of mice treated with the 15-mer peptide, in accordance with the steps outlined in this example.

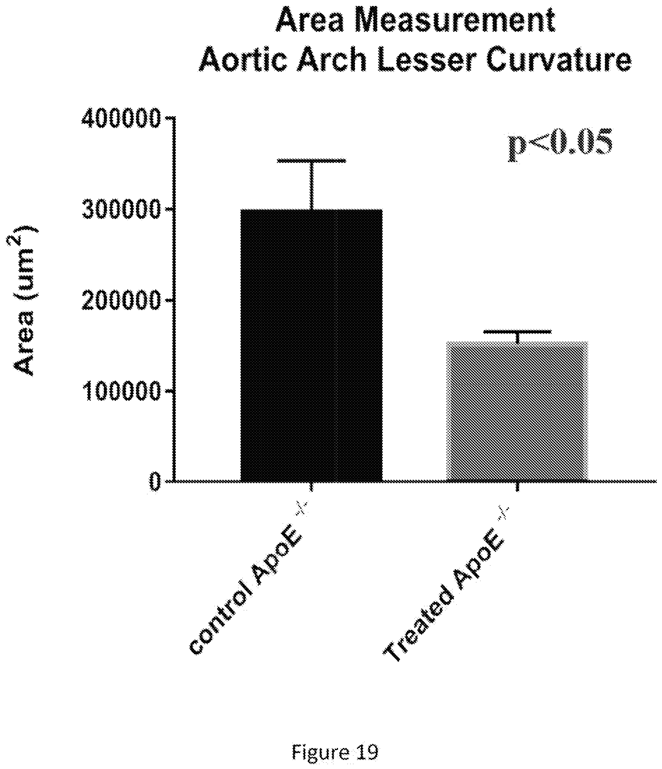

Figure 19 demonstrates the reduction of the total area achieved by peptide treatment. The total area of the 2.5 mm segment (as described in Figure 17) was substantially reduced.

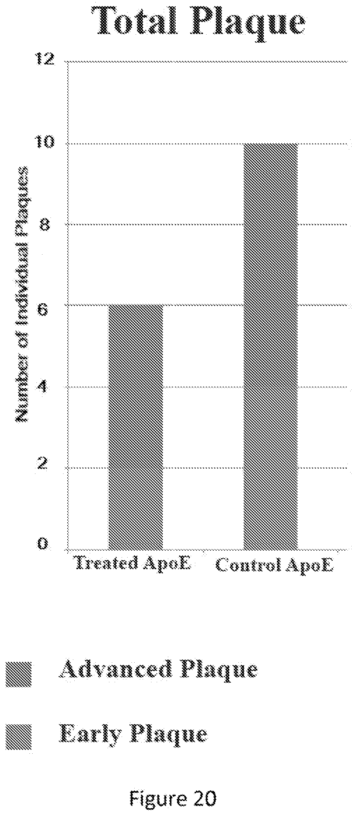

Figure 20 demonstrates the reduction of number of plaque, including early lesions and advanced plaque. The total number of plaque was significantly decreased in the treatment group. Plaque was subdivided based on morphology of early lesions (observable as fatty streaks containing macrophage derived foam cells with varying degrees of lipid accumulation) compared with more advanced fibroatheromas (containing varying degrees of lipid or necrotic core and fibrous caps). All plaque within the designated 2.5mm segment were included. Both the total number and type of plaque were significantly decreased in those subjects treated with the peptide.

This study demonstrates that administration of the peptide abrogates atherosclerosis.

### Example 10

This example demonstrates administration of the KGYY₁₅ (15-mer - SEQ ID NO:7) augments cap formation while reducing advancement of existing disease.

In this study, six ApoE-/- mice received a normal chow diet from 0 to 20 weeks of age. At 20 weeks of age, three mice were randomly assigned to receive dose of 1mg/kg of KGYY₁₅ (15-mer - SEQ ID NO:7) by IV tail injection, once a week for a period of 4 weeks. Control mice received vehicle only. After 4 weeks of treatment, animals were euthanized then perfused through cardiac puncture with 4% paraformaldehyde. Aortic arches were dissected in surcrose gradient and flash frozen. Approximately fifty, 8um longitudinal sections were obtained per mouse. Slides were treated with trichrome stain and analyzed using cellSens software for measurements. Total plaque was measured including 2.5mm lesser curvature and innominate artery.

Figure 20 shows the number of individual early plaques and advanced plaques were reduced in the treated subjects compared to the control subjects.

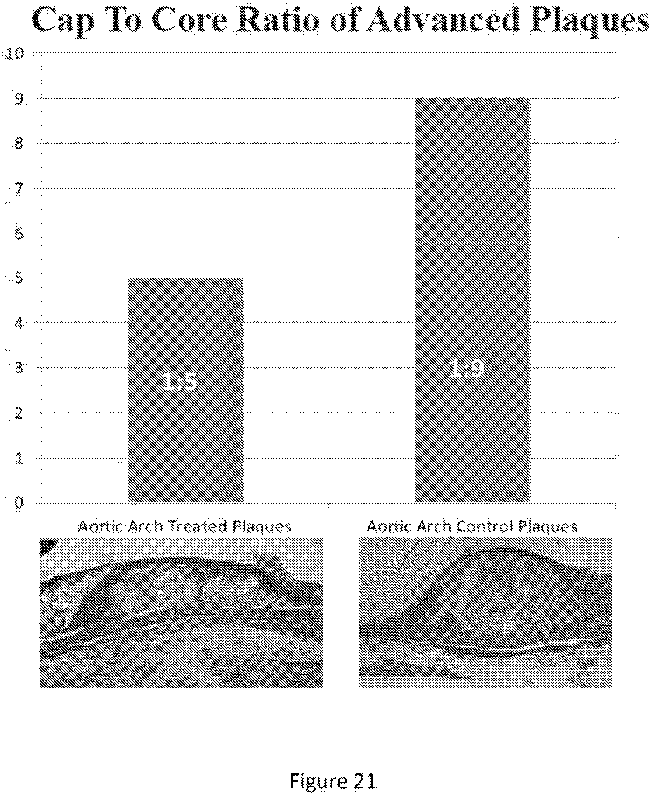

Figure 21 shows that the cap to core ratio of advanced plaques was reduced in the treated subjects compared to the control subjects.

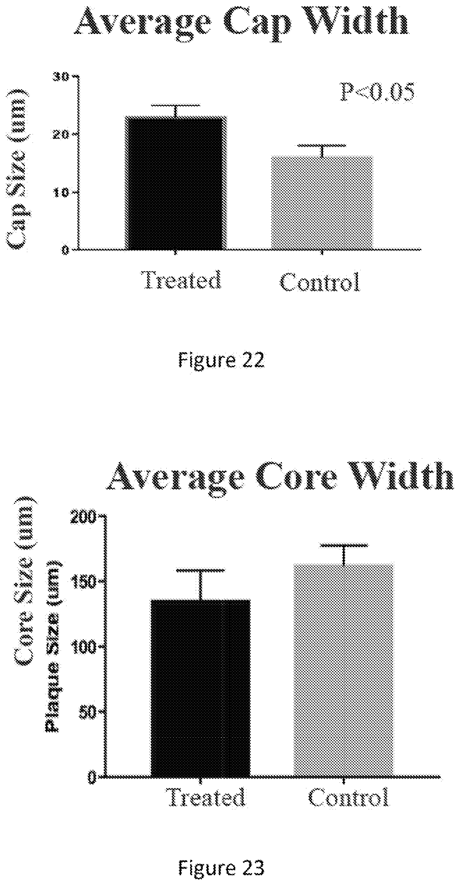

Figure 22 shows the average cap width (cap size) was greater in the treated subjects compared to the control subjects.

Figure 23 shows the average core width (core size/plaque size) was decreased in the subjects treated with the peptide compared to the control subjects.

This example demonstrates that administration of the KGYY₁₅ (15-mer - SEQ ID NO:7) augments cap formation while reducing advancement of existing disease. Moreover, this study further demonstrates that administration of the KGYY₁₅ peptide trends toward results of plaque stability.

From the foregoing, it is readily apparent that T1D shares with atherosclerosis the CD40-CD154 dyad which drives autoimmune inflammation. There are increased Th40 cell levels in peripheral blood of NOD mice, human T1D patients, and ApoE-/- mice. Th40 cells infiltrate the aortic wall and are found within the plaque of ApoE-/- mice. Th40 cells produce the inflammatory cytokine IFNγ at a level greater than that of other cells and this drives inflammation. The KGYY₁₅ peptide targets Th40 cells. The specified peptide furthermore abrogates and modulates atherosclerosis which may be due to Th40 blockade or general blockade of CD40. Moreover, the administration of the specified peptide trends toward more stable plaque types.

### Example 11

Whole human blood was administered the peptide in accordance with similar dosing levels to those used for murine studies.

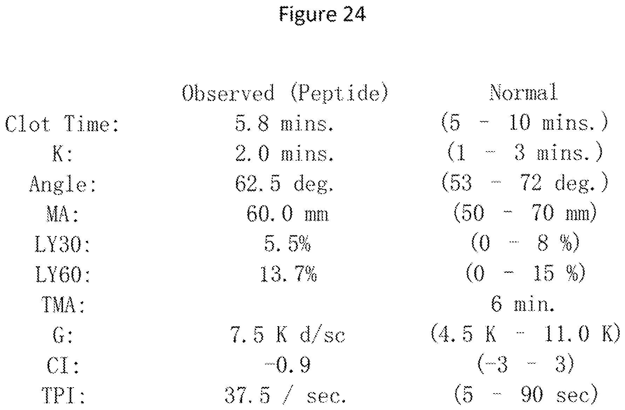

Figure 24 provides the results of clot studies observed in humans.

This study demonstrates that the KGYY₁₅ peptide when administered to humans does not modify or change the clotting of whole human blood significantly outside of normally recognized levels.

### Example 12

This example demonstrates that ApoE mice that have been genetically modified to obtain atherosclerosis and fed a high fat diet and treated with the 6-mer peptide may have the levels of LDL cholesterol values decreased compared to untreated subjects.

In this study, six ApoE-/- mice received a normal chow diet from 0 to 20 weeks of age. At 20 weeks of age, three mice were randomly assigned to receive dose of 1mg/kg of KGYY₆ (6-mer - SEQ ID NO:29) by IV tail injection, once a week for a period of 4 weeks. Control mice received vehicle only.

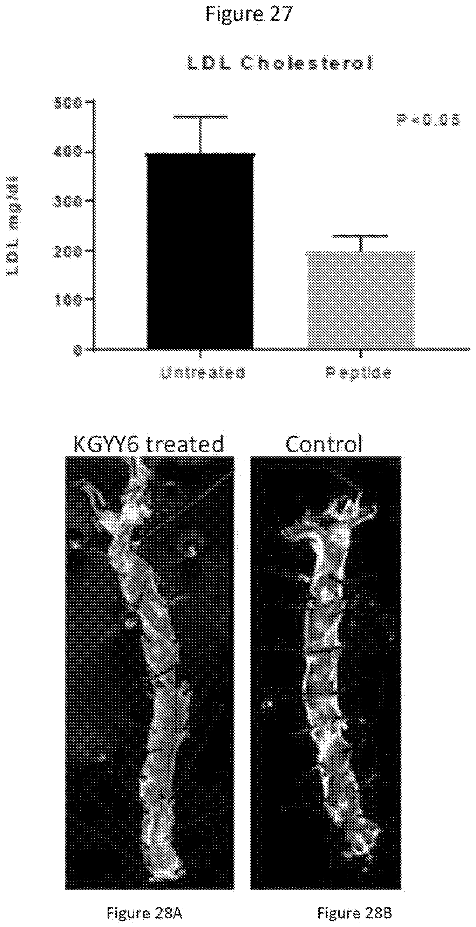

Data obtained from untreated mice and compared to those in treated mice showed statistically significant reduction (>50%) in LDL cholesterol values. This data is provided in Figure 27.

### Example 13

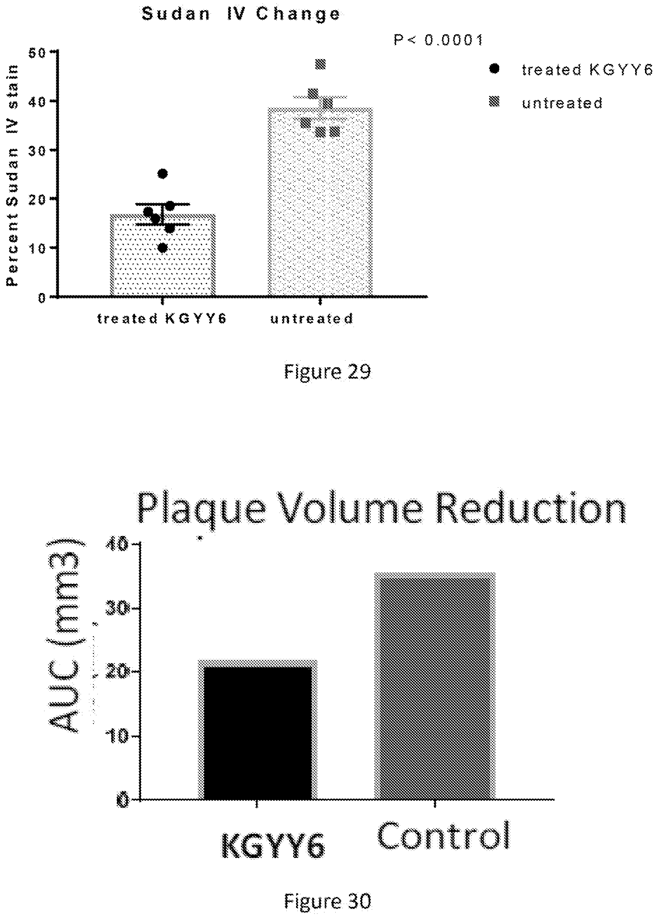

This example demonstrates atherosclerotic changes that ApoE -/- mice experienced when treated with KGYY₆ (6-mer - SEQ ID NO: 29). ApoE -/- mice were fed a high fat diet for 16 weeks. Mice were randomly assigned to receive dose of 1mg/kg of KGYY₆ (6-mer - SEQ ID NO:29) by IV tail injection, once a week for a period of 4 weeks. Control mice received vehicle only. Atherosclerosis was investigated by several methods. En-face analysis utilizing Sudan IV stain (lipid stain) demonstrated a significant reduction in lesion areas. Fig. 28A is an image of KGYY6 treated aortic en-face Sudan IV staining and Fig. 28B is an image of control (untreated) aortic en-face Sudan staining. Fig. 29 is a graph demonstrating the reduction of lesion areas of Sudan IV staining.

Further, measurement of plaque area and morphology was performed using the Paigen method. This method obtains sequential 5µm aortic cross sections from the aortic root beginning at the valve leaflets into the ascending aorta. At 50µm intervals, slides are stained after which the area of atherosclerotic lesion is measured. Individual plaque area measurements are plotted against the micrometer intervals and a curve is established, with the area under the curve (AUC) giving the total volume of plaque. These results are presented in graph format in Fig. 30, which demonstrates that mice treated with KGYY₆ (SEQ ID NO: 29) showed reduction in plaque volume under the curve.

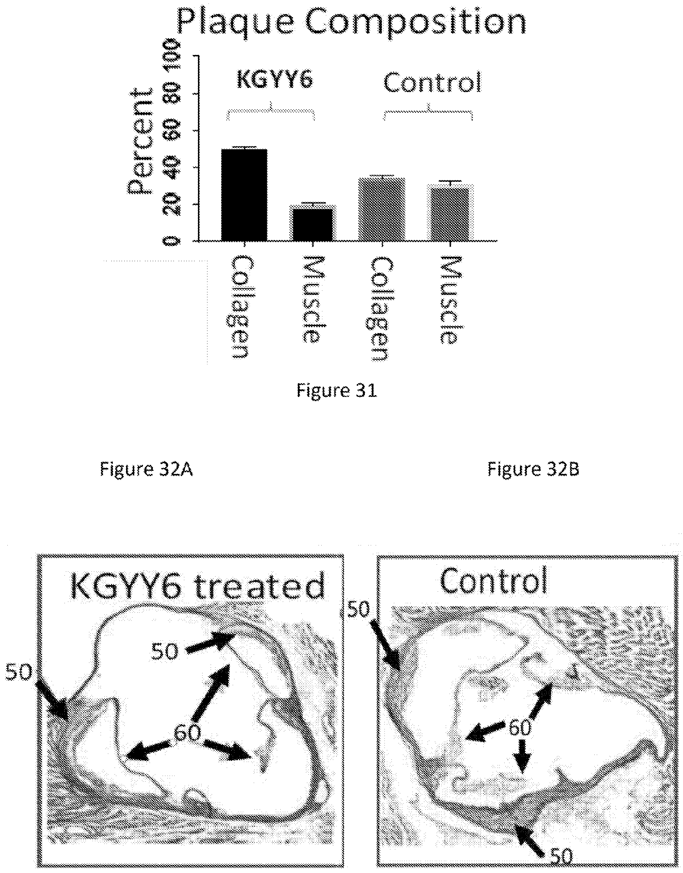

Moreover, characterization of plaque composition or aortic samples was performed using trichrome staining techniques and these results are presented in graph format in Fig. 31. Indeed, this Fig. 31 data, which was generated using Image Pro Plus software analysis, quantifies and shows that plaque compositional changes occurred, including increased collagen and reduced smooth muscle content.

Fig. 32A is an image of trichrome stained cells of the cross-sections of the aorta of the KGYY₆ treated subject. Fig. 32B is an image of trichrome stained cells of the cross-sections of the control subjects. In Fig. 32A, **50** identifies areas characteristic of plaque formation. **60** identifies aortic valve leaflets. Fig. 32B, the control (untreated) **50** areas characteristic of plaque (area under the curve) are greater than those in the subjects treated with the KGYY₆ peptide.

### Example 15

This example demonstrates that KGYY₆ (6-mer - SEQ ID NO:29) results in significant improvement in glucose tolerance and insulin sensitivity.

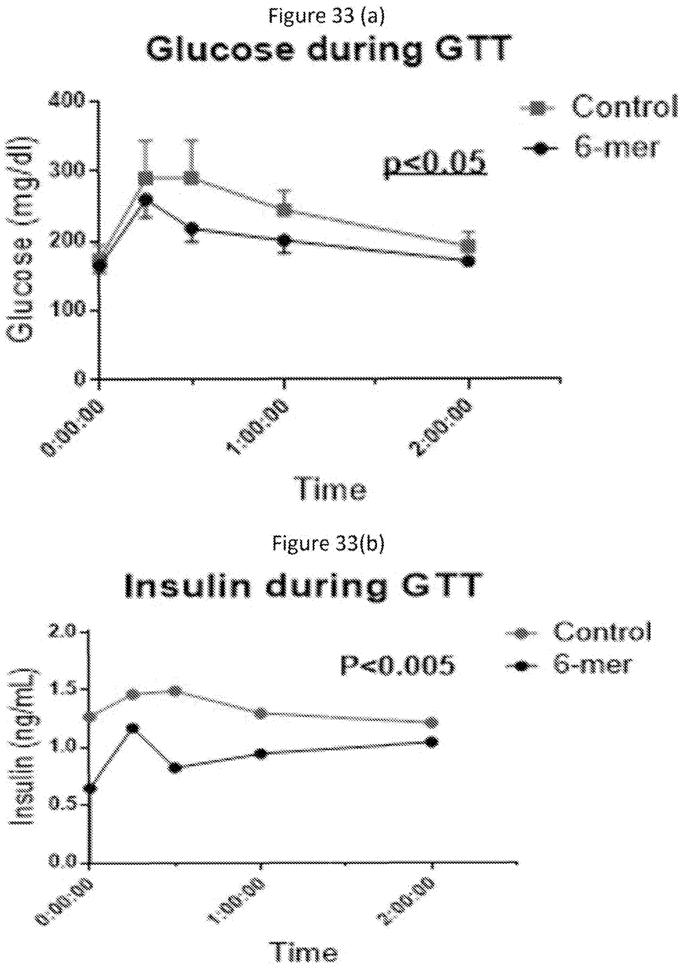

ApoE -/- mice were fed a 60% high fat diet (research diet) for 1 week. A select population of mice were then injected with 6-mer peptide at a dose of 1mg/kg and others were untreated and tracked as controls. Glucose tolerance testing (GTT) was performed by fasting 6 hours, followed by intraperitoneal injection of 1g/kg body weight glucose in water. Both blood glucose and blood serum insulin were measured at 0, 15 minutes, 30 minutes, 60 minutes, and 2 hours. The results of this study are shown in Figs. 33(a) and 33(b). Peptide treated ApoE deficient mice demonstrated significantly increased glucose tolerance as well as significantly improved insulin sensitivity and lowered plasma insulin levels compared to control.

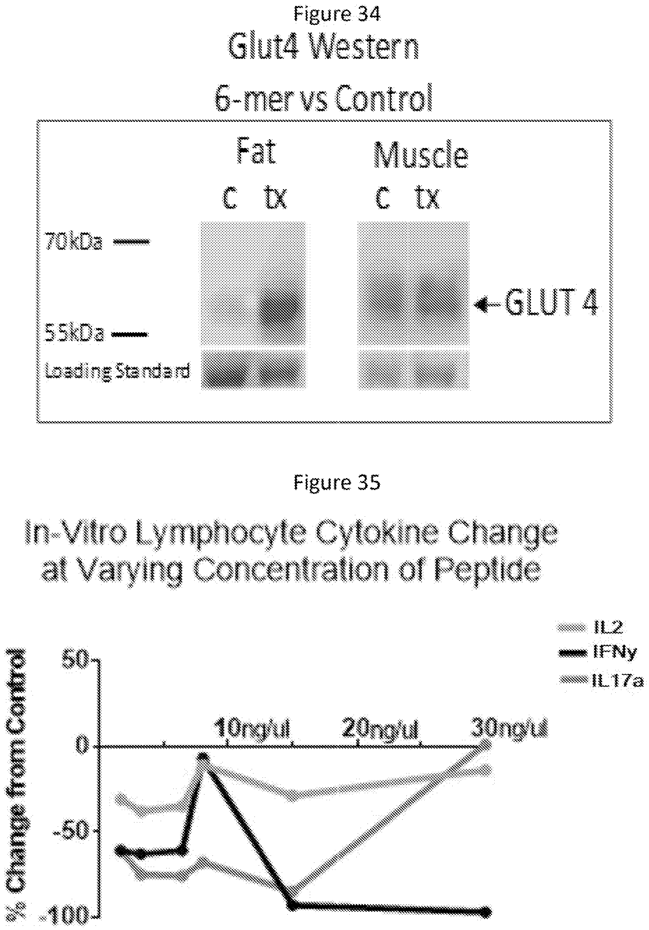

Western analysis was performed on adipose and muscle tissue of the treated and untreated mice, demonstrating an increase in expression of the glucose transport protein (GLUT4) in white adipose tissue, as shown in Fig. 34. Moreover, the western analysis also demonstrated that the muscle tissue showed increased expression of the GLUT4 protein. GLUT4 is responsible for glucose uptake in response to insulin and known to have reduced expression in type 2 diabetes.

This study demonstrates that administration of the peptide affects glucose tolerance, insulin sensitivity, and GLUT4 levels, each of which may be important to treating type 2 diabetic subjects.

### Example 16

This example demonstrates administration of the KGYY₆ (6-mer - SEQ ID NO:29) affects the inflammatory cytokine production of IL2, INFγ, and IL17a.

Spleens from ApoE mice and C57BL/6 mice were processed for lymphocytes. ApoE -/- and C57BL/6 mice were fed a 60% high fat diet (research diet) for 1 week. Splenic lymphocytes were treated with 6-mer in vitro for 24 hours. Cells were placed in media overnight in the presence of varying concentrations of 6-mer peptides. The following morning, Brefeldin A was administered for 4 hours. All cells were stained for CD3, CD4, CD40 (Th40 cells) and measured by use of flow cytometry for their production of IL2, INFγ, and IL17a. The results of this study are shown in Fig. 35.

## Claims

1. A peptide that comprises an amino acid sequence of SEQ ID NO: 3 for use in a method for preventing, reducing, treating, and/or reversing type 2 diabetes in a subject, the method comprising administering to the subject an effective amount of the peptide, wherein the peptide is no more than 25 amino acids in length and that affects the interaction of CD40 with CD154/CD40-ligand by inhibiting the binding of CD40 to CD154.

2. The peptide for use according to any of the prior claims, wherein administering said peptide affects the interaction of CD40 with CD154 in such a manner as to prevent the expansion of Th40 cells, optionally wherein the number of Th40 cells in the patient is reduced.

3. The peptide for use according to any of the prior claims, wherein administering said peptide affects the interaction of CD40 with CD154 in such a manner as to alter the cytokine expression profile of a cell population in the subject to whom said peptide is administered.

4. The peptide for use according to any of the prior claims, wherein said peptide comprises an amino acid sequence selected from the group of SEQ ID NO:4, SEQ ID NO:5, SEQ ID NO:6, SEQ ID NO:8, SEQ ID NO:9 and SEQ ID NO:27.

5. The peptide for use according to any of the prior claims, wherein said peptide consists of an amino acid sequence selected from the group of SEQ ID NO:4-9 and 24-30.

6. The peptide for use according to any of the prior claims, wherein following administration of the peptide the subject does not produce autoantibodies responsive to the peptide and/or CD154 and/or GLUT 4, and optionally wherein the subject's T cells do not elicit T cell antigen recall.

7. The peptide for use according to any of the prior claims, wherein the peptide is administered using a delivery method selected from intramuscular (IM) delivery, intravenous (IV) delivery, subcutaneous (SC) delivery, oral delivery, gavage delivery, emollient/skin delivery, or transdermal patch.

8. The peptide for use according to any of the prior claims, wherein the peptide is administered using an extended delivery method selected from an implantable device, a hydrophilic polymer formulation, a permeable polymeric membrane, injectable gel implants, solvent extraction system, phase inversion system, thermosensitive gels, pH dependent *in situ* gels, microparticles, microspheres, nanoparticles, nanospheres, bio-degradable implants, or photoactivated depot.

9. The peptide for use according to any of the prior claims, wherein the peptide is administered in an amount sufficient to reduce or inhibit interferon-γ (IFN-γ), interleukin-2 (IL-2) and/or interleukin-17 (IL-17) signaling, wherein, the INF-γ, IL-2 and/or IL-17 signaling is associated with type 2 diabetes in the subject.

10. The peptide for use according to any of the prior claims, wherein the subject has type 2 diabetes.

11. The peptide for use according to claim 10, wherein a therapeutically effective amount of the peptide is administered.

## Patentansprüche

1. Ein Peptid, das eine Aminosäuresequenz der SEQ ID NO: 3 zur Verwendung in einem Verfahren zur Prävention, Reduktion, Behandlung und/oder Umkehr von Typ-2-Diabetes bei einem Patienten umfasst, wobei das Verfahren das Verabreichen einer wirksamen Menge des Peptids an den Patienten umfasst, wobei das Peptid nicht mehr als 25 Aminosäuren lang ist und die Wechselwirkung von CD40 mit dem CD154/CD40-Liganden durch Hemmung der Bindung von CD40 an CD154 beeinflusst.

2. Das Peptid zur Verwendung nach einem der vorhergehenden Ansprüche, wobei die Verabreichung des Peptids die Wechselwirkung von CD40 mit CD154 auf eine solche Weise beeinflusst, dass die Vermehrung von Th40-Zellen verhindert wird, wobei gegebenenfalls die Anzahl der Th40-Zellen in dem Patienten verringert wird.

3. Das Peptid zur Verwendung nach einem der vorhergehenden Ansprüche, wobei die Verabreichung des Peptids die Wechselwirkung von CD40 mit CD154 auf eine solche Weise beeinflusst, dass das Zytokinexpressionsprofil einer Zellpopulation in dem Patienten, dem das Peptid verabreicht wird, verändert wird.

4. Das Peptid zur Verwendung nach einem der vorhergehenden Ansprüche, wobei das Peptid eine Aminosäuresequenz umfasst, die aus der Gruppe von SEQ ID NO:4, SEQ ID NO:5, SEQ ID NO:6, SEQ ID NO:8, SEQ ID NO:9 und SEQ ID NO:27 ausgewählt ist.

5. Das Peptid zur Verwendung nach einem der vorhergehenden Ansprüche, wobei das Peptid aus einer Aminosäuresequenz besteht, die aus der Gruppe von SEQ ID NO:4-9 und 24-30 ausgewählt ist.

6. Das Peptid zur Verwendung nach einem der vorhergehenden Ansprüche, wobei der Patient nach der Verabreichung des Peptids keine Autoantikörper produziert, die auf das Peptid und/oder CD154 und/oder Glut 4 ansprechen, und wobei gegebenenfalls die T-Zellen des Patienten kein T-Zell-Antigen-Recall (Wiedererkennung) hervorrufen.

7. Das Peptid zur Verwendung nach einem der vorhergehenden Ansprüche, wobei das Peptid unter Verwendung eines Abgabeverfahrens verabreicht wird, das ausgewählt ist aus intramuskulärer (IM) Abgabe, intravenöser (IV) Abgabe, subkutaner (SC) Abgabe, oraler Abgabe, Gavage-Abgabe, Emolument/Hautabgabe oder transdermalem Pflaster.

8. Das Peptid zur Verwendung nach einem der vorhergehenden Ansprüche, wobei das Peptid unter Verwendung eines verlängerten Abgabeverfahrens verabreicht wird, das aus einer implantierbaren Vorrichtung, einer hydrophilen polymeren Formulierung, einer durchlässigen polymeren Membran, injizierbaren Gelimplantaten, einem Lösungsmittelextraktionssystem, einem Phaseninversionssystem, wärmeempfindlichen Gelen, pH-abhängigen *in situ-*Gelen*,* Mikropartikeln, Mikrosphären, Nanopartikeln, Nanosphären, biologisch abbaubaren Implantaten oder einem photoaktivierten Depot ausgewählt ist.

9. Das Peptid zur Verwendung nach einem der vorhergehenden Ansprüche, wobei das Peptid in einer Menge verabreicht wird, die ausreicht, um die Interferon-γ (IFN-γ)-, Interleukin-2 (IL-2)- und/oder Interleukin-17 (IL-17)-Signalisierung zu reduzieren oder zu hemmen, wobei die INF-γ-, IL-2- und/oder IL-17-Signalisierung bei dem Patienten in Zusammenhang mit Typ-2-Diabetes steht.

10. Das Peptid zur Verwendung nach einem der vorhergehenden Ansprüche, wobei der Patient Typ-2-Diabetes hat.

11. Das Peptid zur Verwendung nach Anspruch 10, wobei eine therapeutisch wirksame Menge des Peptids verabreicht wird.

## Revendications

1. Un peptide qui comprend une séquence d'acides aminés de la SEQ ID NO: 3 pour son utilisation dans un procédé pour prévenir, réduire, traiter et/ou inverser le diabète de type 2 chez un sujet, le procédé comprenant l'administration au sujet d'une quantité efficace du peptide, dans lequel le peptide est d'une longueur qui n'est pas supérieure à 25 acides aminés et qui affecte l'interaction de CD40 avec le ligand de CD40/CD154 en inhibant la liaison de CD40 à CD154.

2. Le peptide pour l'utilisation selon l'une quelconque des revendications précédentes, dans lequel l'administration dudit peptide affecte l'interaction de CD40 avec CD154 de manière à empêcher la prolifération de cellules Th40, éventuellement dans lequel le nombre de cellules Th40 chez le patient est réduit.

3. Le peptide pour l'utilisation selon l'une quelconque des revendications précédentes, dans lequel l'administration dudit peptide affecte l'interaction de CD40 avec CD154 de manière à modifier le profil d'expression de cytokines d'une population cellulaire chez le sujet auquel ledit peptide est administré.

4. Le peptide pour l'utilisation selon l'une quelconque des revendications précédentes, dans lequel ledit peptide comprend une séquence d'acides aminés choisie dans le groupe de SEQ ID NO: 4, SEQ ID NO: 5, SEQ ID NO: 6, SEQ ID NO : 8, SEQ ID NO : 9 et SEQ ID NO : 27.

5. Le peptide pour l'utilisation selon l'une quelconque des revendications précédentes, dans lequel ledit peptide consiste en une séquence d'acides aminés choisie dans le groupe des SEQ ID NO: 4-9 et 24-30.

6. Le peptide pour l'utilisation selon l'une quelconque des revendications précédentes, dans lequel, après l'administration du peptide, le sujet ne produit pas d'auto-anticorps répondant au peptide et/ou à CD154 et/ou à GLUT 4, et éventuellement dans lequel les cellules T du sujet ne suscitent pas de *recall* (reconnaissance) d'antigènes de cellules T.

7. Le peptide pour l'utilisation selon l'une quelconque des revendications précédentes, dans lequel le peptide est administré en utilisant un procédé de livraison choisi parmi la livraison intramusculaire (IM), la livraison intraveineuse (IV), la livraison sous-cutanée (SC), la livraison orale, la livraison par gavage, la livraison d'emolument/dermique ou le timbre transdermique.

8. Le peptide pour l'utilisation selon l'une quelconque des revendications précédentes, dans lequel le peptide est administré en utilisant un procédé de livraison prolongée choisi parmi un dispositif implantable, une formulation polymère hydrophile, une membrane polymère perméable, des implants en gel injectables, un système d'extraction par solvant, un système d'inversion de phase, des gels thermosensibles, des gels *in situ* dépendants du pH, des microparticules, des microsphères, des nanoparticules, des nanosphères, des implants biodégradables ou un dépôt photoactivé.

9. Le peptide pour l'utilisation selon l'une quelconque des revendications précédentes, dans lequel le peptide est administré en une quantité suffisante pour réduire ou inhiber la signalisation de l'interféron-γ (IFN-γ), de l'interleukine-2 (IL-2) et/ou de l'interleukine-17 (IL-17), dans lequel la signalisation de l'INF-y, de l'IL-2 et/ou de l'IL-17 est associée au diabète de type 2 chez le sujet.

10. Le peptide pour l'utilisation selon l'une quelconque des revendications précédentes, dans lequel le sujet a un diabète de type 2.

11. Le peptide pour l'utilisation selon la revendication 10, dans lequel une quantité thérapeutiquement efficace du peptide est administrée.
